# EUROPEAN PATENT APPLICATION

(11) **EP 4 458 833 A1**
(43) Date of publication of application: **06.11.2024**
(21) Application number: 22916140.1
(22) Date of filing: 27.12.2022
(51) Int. Cl.: C07F 9/6533, C07H 21/04

(54) **METHOD FOR PRODUCING OLIGONUCLEIC ACID COMPOUND**

(30) Priority: 27.12.2021 JP 2021212132
(71) Applicant: Nippon Shinyaku Co., Ltd., Kyoto-shi Kyoto 601-8550 (JP)
(72) Inventor: NOGATA, Masaki, Kyoto-shi, Kyoto 601-8550 (JP); SHIBA, Yoshinobu, Kyoto-shi, Kyoto 601-8550 (JP); SHIMOOKA, Takuya, Kyoto-shi, Kyoto 601-8550 (JP); YOKOI, Kento, Kyoto-shi, Kyoto 601-8550 (JP)
(74) Representative: Vossius & Partner Patentanwälte Rechtsanwälte mbB
(86) International application number: PCT/JP2022/048385
(87) International publication number: WO 2023/127918

(57) **Abstract**

The present invention relates to a method for producing a morpholino nucleic acid oligomer (each symbol is as defined in the description), the method including a step for treating a compound of formula (II) using a solution that contains an acid and a scavenger in an elongation reaction of a morpholino nucleic acid oligomer represented by the following formula and removing R¹ from the compound of formula (II) to produce a compound of formula (III).

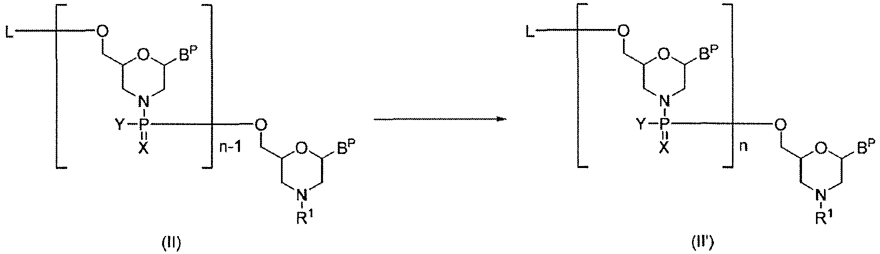

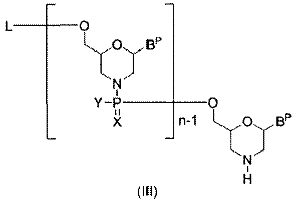

## Description

### [TECHNICAL FIELD]

The present invention relates to a novel preparation method for an oligonucleic acid compound.

### [BACKGROUND ART]

A solid-phase method and a liquid-phase method are known as methods for preparing an oligonucleic acid compound. The solid-phase method is a heterogeneous reaction method in which a nucleic acid is extended while a substrate supported on a solid-phase support is brought into contact with a solution containing a reaction reagent. In the solid-phase method, a so-called batch method is used in which a reaction vessel with a filter is used and a reaction is carried out in the vessel (see, for example, NON-PATENT DOCUMENT 1 and PATENT DOCUMENT 1). In addition, a pseudo-flow synthesis method is also known in which, as in an automatic nucleic acid synthesizer (e.g., DNA/RNA synthesizer), a solid-phase support is placed in a column and a solution containing a reaction reagent is passed through the column to cause a reaction.

Meanwhile, the liquid-phase method is a homogeneous reaction method in which a nucleic acid is extended by causing a reaction in a solution containing both a substrate and a reaction reagent. In the liquid-phase method as well, a batch method in which a reaction is carried out in a vessel is used (see, for example, PATENT DOCUMENT 2 and PATENT DOCUMENT 3).

In any of the cases of the solid-phase method, the liquid-phase method, the batch method, and the pseudo-flow synthesis method, in a chemical synthesis method for an oligonucleic acid compound, a nucleic acid is extended by repeating many times a "deprotection" reaction for removing a protective group for an oxygen atom or amino group on a nucleic acid compound, and a "condensation" reaction for forming a bond between a phosphorus atom and an oxygen atom or nitrogen atom deprotected to be enabled to react.

In the case of using an oligonucleic acid compound as a raw material for a pharmaceutical product, since the oligonucleic acid compound is required to have a high purity, it is necessary to improve the reaction efficiency and the reaction rate while suppressing the formation of by-products in each step of an elongation reaction. However, this is difficult since solvents and reagents that can be used are limited.

### [PRIOR ART DOCUMENTS]

### [PATENT DOCUMENTS]

[PATENT DOCUMENT 1] WO1991/09033A1
[PATENT DOCUMENT 2] WO2014/077292A1
[PATENT DOCUMENT 3] WO2013/122236A1

### [NON-PATENT DOCUMENTS]

[NON-PATENT DOCUMENT 1] Acc. Chem. Res., Vol. 24, 278-284, 1991

### [SUMMARY OF INVENTION]

### [PROBLEMS TO BE SOLVED BY THE INVENTION]

In the elongation reaction of the oligonucleic acid compound, deprotection and condensation reactions are repeated, thus resulting in complex impurities. An object of the present invention is to provide a novel preparation method that identifies impurities affecting the purity of an oligonucleic acid compound and controls the generation of such impurities.

### [MEANS OF SOLVING THE PROBLEMS]

By diligently examining the treatment of reaction raw materials before condensation and intermediates in an elongation reaction of an oligonucleic acid compound, the present inventors have found that a deprotection reaction and a condensation reaction proceed efficiently, and further that impurities produced by pretreatment of the condensation reaction affect the purity of the oligonucleic acid compound, and have achieved the present invention.

That is, the present invention relates to the following.
<1> A preparation method for a morpholino nucleic acid oligomer, including, in an elongation reaction of the morpholino nucleic acid oligomer: 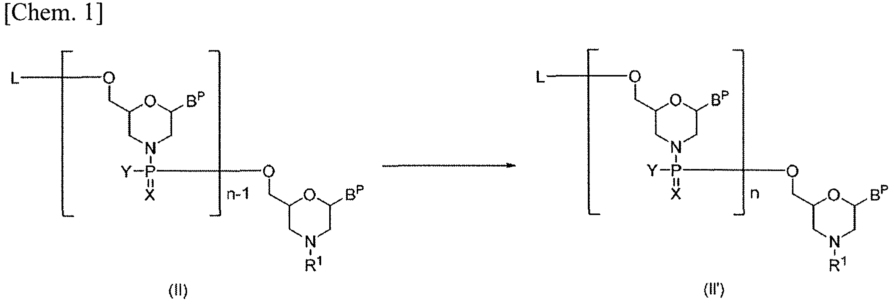 [wherein
   each B^{P} is independently an optionally protected nucleic acid base,
   R¹ is trityl, monomethoxytrityl, or dimethoxytrityl,
   X is O or S,
   Y is dialkylamino or alkoxy,
   n is any integer in a range of 1 to 99, preferably any integer in a range of 15 to 30, and further preferably any integer in a range of 18 to 28, and
   L is hydrogen, acyl, or a group represented by formula (IV): 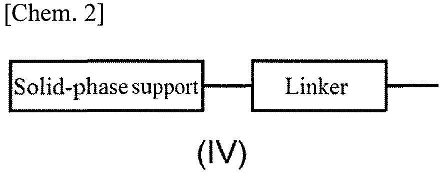 ],
   a step of treating a compound of formula (II):

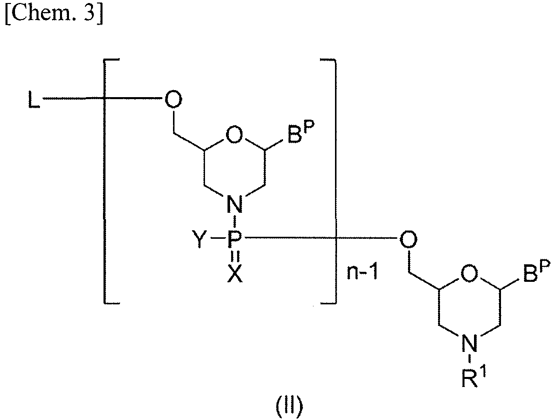

   [wherein L, B^{P}, X, Y, R¹, and n are as described above]
   with a solution containing an acid and a scavenger, to remove R¹ from the compound of formula (II) to obtain a compound of formula (III): 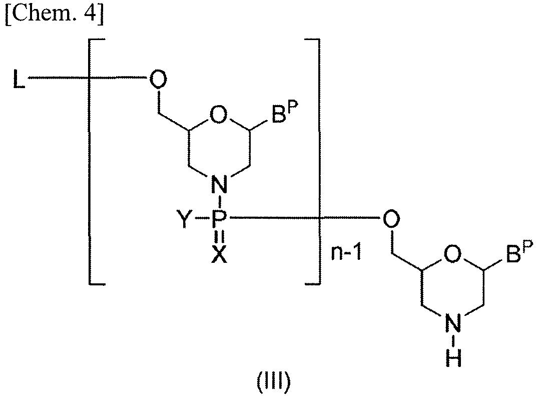
   [wherein L, B^{P}, X, Y, and n are as described above].
<2> The preparation method according to <1>, further including a step of treating the compound of formula (III): 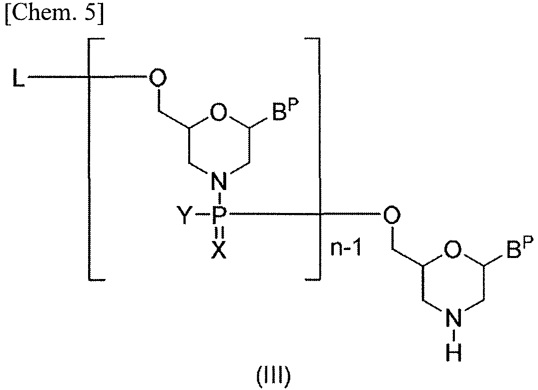
   [wherein L, B^{P}, X, Y, and n are as described above]
   with a solution containing a base, an alcohol, and a halogen solvent.
<3> The preparation method according to <1> or <2>, further including a step of treating the compound of formula (III): 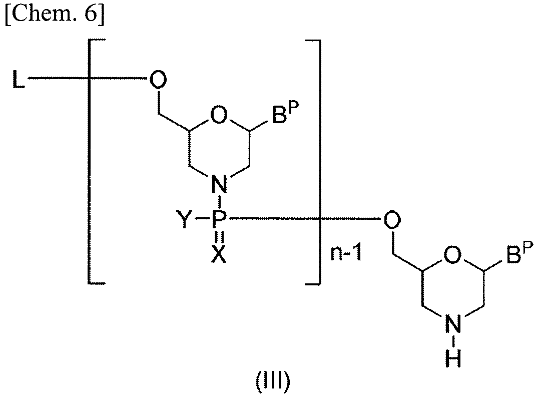
   [wherein L, B^{P}, X, Y, and n are as described above]
   with a solution containing an organic amine and an aprotic polar solvent.
<4> The preparation method according to any one of <1> to <3>, further including a step of treating a compound of formula (VIII): 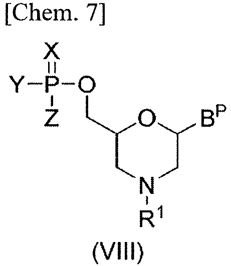
   [wherein B^{P}, X, Y, and R¹ are as described above, and Z is a halogen (e.g., chloro, bromo, iodo)]
   with a solution containing an organic amine and an aprotic polar solvent.
<5> The preparation method according to any one of <1> to <4>, further including a step of reacting the compound of formula (III): 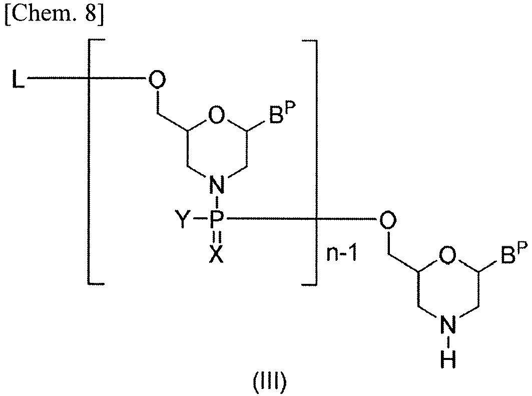
   [wherein L, B^{P}, X, Y, and n are as described above]
   in the presence of an organic amine with a compound of formula (VIII): 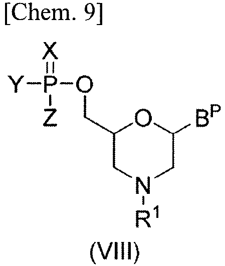
   [wherein X, Y, Z, B^{P}, and R¹ are as described above]
   to obtain a compound of formula (II'): 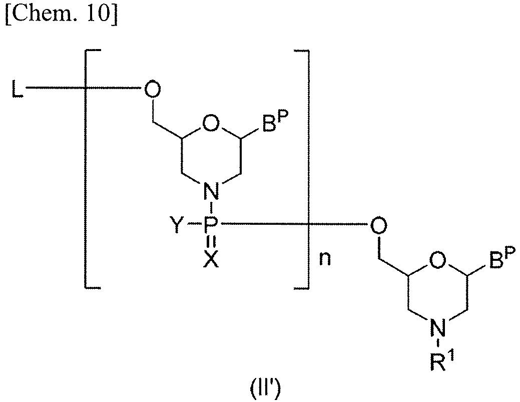
   [wherein L, B^{P}, X, Y, R¹, and n are as described above].
<6> The preparation method according to any one of <1> to <5>, further including a step of treating a compound of formula (II'): 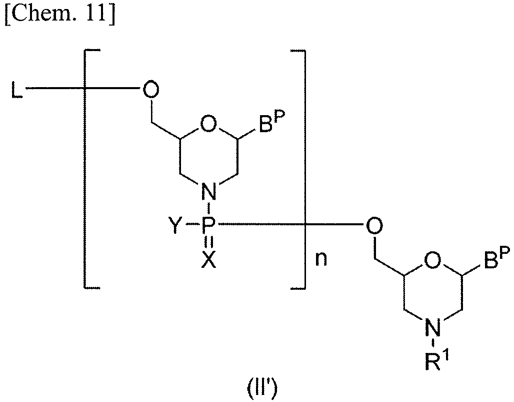
   [wherein L, B^{P}, X, Y, R¹, and n are as described above]
   with a solution containing an alcohol and/or a halogen solvent.
<7> A preparation method for a morpholino nucleic acid oligomer, including, in an elongation reaction of the morpholino nucleic acid oligomer: 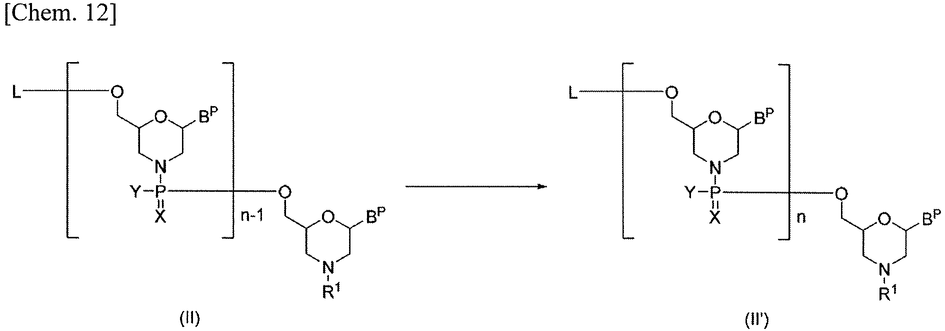 [wherein
   each B^{P} is independently an optionally protected nucleic acid base,
   R¹ is trityl, monomethoxytrityl, or dimethoxytrityl,
   X is O or S,
   Y is dialkylamino or alkoxy,
   n is any integer in a range of 1 to 99, preferably any integer in a range of 15 to 30, and further preferably any integer in a range of 18 to 28, and
   L is hydrogen, acyl, or a group represented by formula (IV): 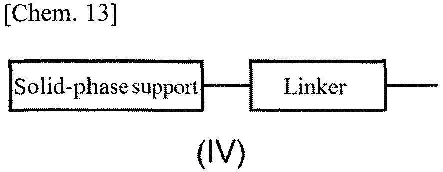 ]
   a step of treating a compound of formula (II):

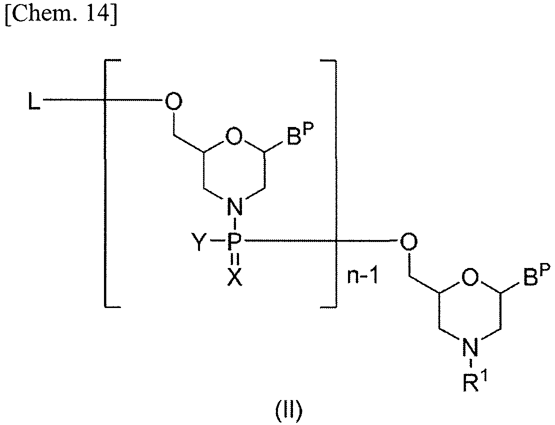

   [wherein L, B^{P}, X, Y, R¹, and n are as described above]
   with a solution containing an acid and a scavenger, to remove R¹ from the compound of formula (II) to obtain a compound of formula (III): 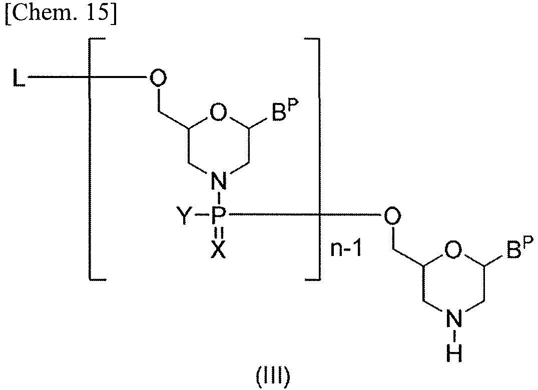
   [wherein L, B^{P}, X, Y, and n are as described above],
      a step of treating the compound of formula (III): 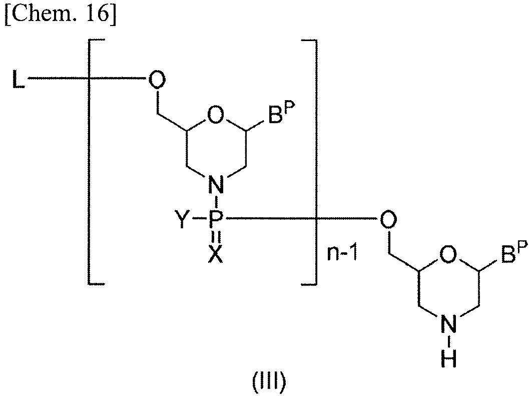
   [wherein L, B^{P}, X, Y, and n are as described above]
   with a solution containing an organic amine and an aprotic polar solvent, and
      a step of treating a compound of formula (VIII): 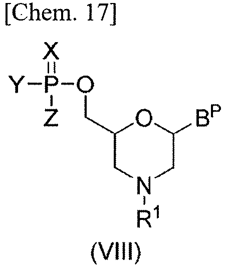
   [wherein B^{P}, X, Y, Z, and R¹ are as described above]
   with a solution containing an organic amine and an aprotic polar solvent.
<8> A preparation method for a morpholino nucleic acid oligomer, including, in an elongation reaction of the morpholino nucleic acid oligomer: 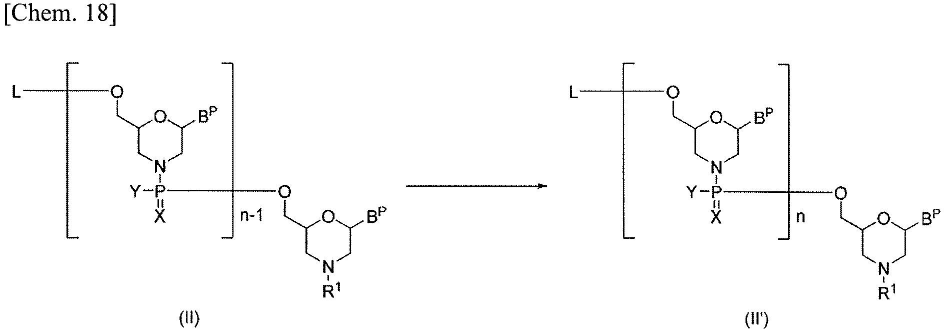 [wherein
   each B^{P} is independently an optionally protected nucleic acid base,
   R¹ is trityl, monomethoxytrityl, or dimethoxytrityl,
   X is O or S,
   Y is dialkylamino or alkoxy,
   n is any integer in a range of 1 to 99, preferably any integer in a range of 15 to 30, and further preferably any integer in a range of 18 to 28, and
   L is hydrogen, acyl, or a group represented by formula (IV): 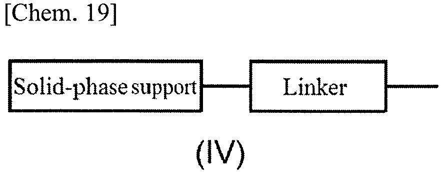 ],
   a step of treating a compound of formula (II):

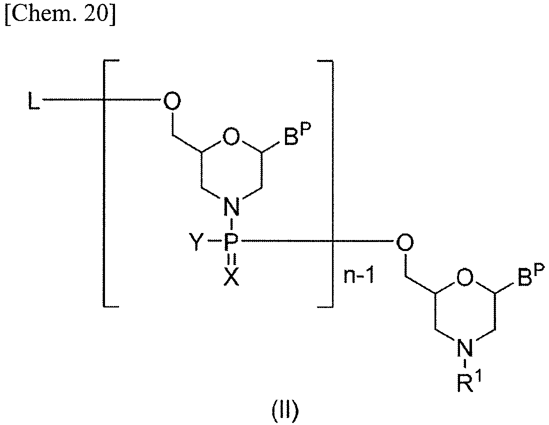

   [wherein L, B^{P}, X, Y, R¹, and n are as described above]
   with a solution containing an acid and a scavenger, to remove R¹ from the compound of formula (II) to obtain a compound of formula (III): 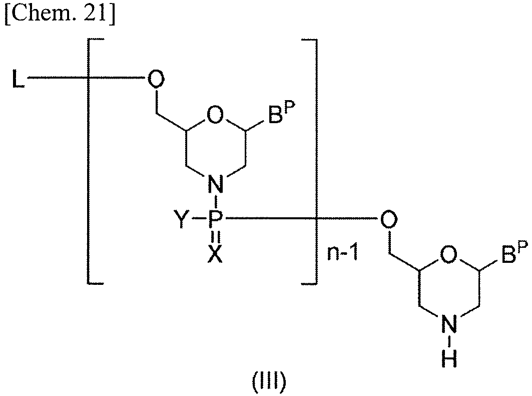
   [wherein L, B^{P}, X, Y, and n are as described above], and
      a step of treating the compound of formula (III): 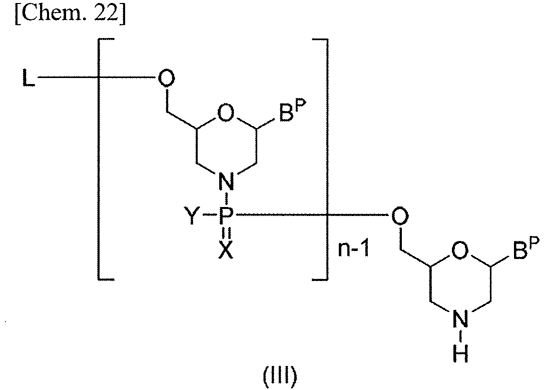
   [wherein L, B^{P}, X, Y, and n are as described above]
   and a compound of formula (VIII): 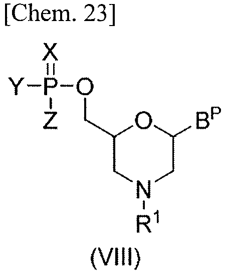
   [wherein B^{P}, X, Y, Z, and R¹ are as described above]
   with a solution containing an organic amine and an aprotic polar solvent.
<9> The preparation method for the morpholino nucleic acid oligomer according to any one of <1> to <8>, wherein the solution containing the acid and the scavenger is a solution containing trifluoroacetic acid and triisopropylsilane.
<10> The preparation method for the morpholino nucleic acid oligomer according to any one of <1> to <9>, wherein the solution containing the acid and the scavenger is a solution containing trifluoroacetic acid, triethylamine, triisopropylsilane, 2,2,2-trifluoroethanol, and dichloromethane.
<11> The preparation method for the morpholino nucleic acid oligomer according to any one of <1> to <9>, wherein the solution containing the organic amine and the aprotic polar solvent is N-ethylmorpholine and 1,3-dimethyl-2-imidazolidinone.
<12> The preparation method according to any one of <1> to <11>, wherein the solid-phase support is a swellable polystyrene, a non-swellable polystyrene, a PEG chain-attached polystyrene, controlled pore glass, oxalylized controlled pore glass, a TentaGel support-amino polyethylene glycol-derivatized support, or a copolymer of Poros-polystyrene/divinylbenzene.
<13> The preparation method according to any one of <1> to <12>, wherein the linker is short-chain alkylene, long-chain alkylene, amino-short-chain alkylene, amino-long-chain alkylene, diacyl short-chain alkylene (e.g., succinyl), diacyl long-chain alkylene, or dialkylene sulfonyl.

### [EFFECT OF INVENTION]

The present invention enables to prepare a morpholino nucleic acid oligomer economically with a good yield and with high purity.

### [DESCRIPTION OF EMBODIMENTS]

Hereinafter, the present invention is described in detail.

### <Morpholino nucleic acid oligomer>

In one aspect of the present invention, a morpholino nucleic acid oligomer is an oligomer having, as a constituent unit, a group represented by a formula: 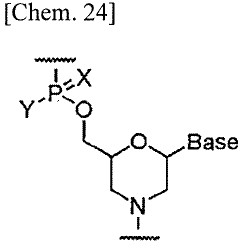 [wherein Base represents a nucleic acid base, and X and Y are as described above].

### <Nucleic acid base>

In one aspect of the present invention, examples of the "nucleic acid base" include adenine, guanine, hypoxanthine, cytosine, thymine, uracil, and modified bases thereof. Examples of such modified bases include, but are not limited to, pseudouracil, 3-methyluracil, dihydrouracil, 5-alkylcytosines (e.g., 5-methylcytosine), 5-alkyluracils (e.g., 5-ethyluracil), 5-halouracils (e.g., 5-bromouracil, 5-fluorouracil), 6-azapyrimidine, 6-alkylpyrimidines (e.g., 6-methyluracil), 2-thiouracil, 4-thiouracil, 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N⁶-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N⁶-isopentenyladenine, uracil-5-oxyacetic acid, 2-thiocytosine, purine, 2,6-diaminopurine, 2-aminopurine, isoguanine, indole, imidazole, and xanthine. The amino group or hydroxyl group of the nucleic acid base for Base may be protected.

### <Preparation method for morpholino nucleic acid oligomer>

One aspect of the present invention is a preparation method for a morpholino nucleic acid oligomer (hereinafter referred to as morpholino nucleic acid oligomer (I)) of formula (I): 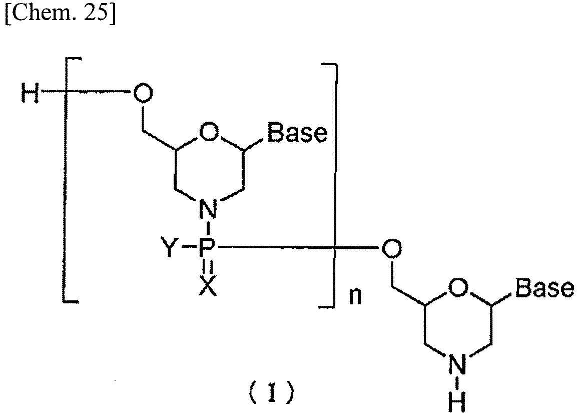 [wherein
Base, X, and Y are as defined above, and
n is any integer in a range of 1 to 99, preferably any integer in a range of 15 to 30, and further preferably any integer in a range of 18 to 28].

One aspect of the present invention is a preparation method for a morpholino nucleic acid oligomer, including an elongation reaction of the morpholino nucleic acid oligomer in which a compound of formula (II) and a compound of formula (VIII) are reacted to obtain a compound of formula (II'): 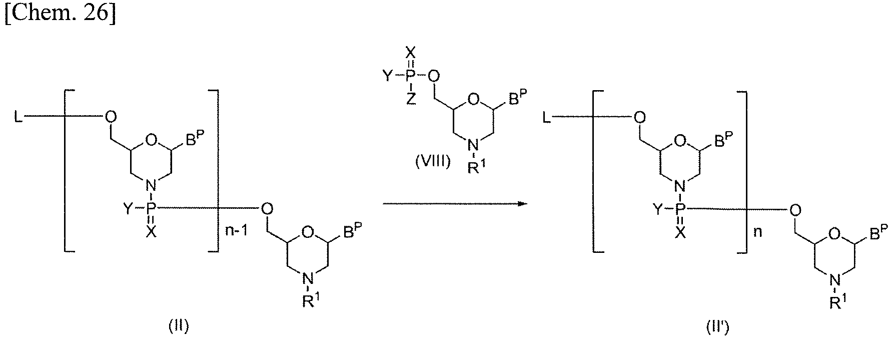 [wherein
each B^{P} is independently an optionally protected nucleic acid base,
R¹ is trityl, monomethoxytrityl, or dimethoxytrityl,
X is O or S,
Y is OH, lower alkoxy, mono-lower alkylamino, or di-lower alkylamino,
Z is a halogen (e.g., chloro, bromo, iodo),
n is any integer in a range of 1 to 99, preferably any integer in a range of 15 to 30, and further preferably any integer in a range of 18 to 28, and
L is hydrogen, acyl, or a group represented by formula (IV): 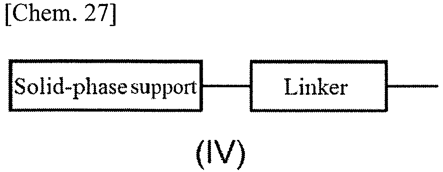 ].

In one aspect of the present invention, the optionally protected nucleic acid base includes, for example, both unprotected "nucleic acid bases" and protected "nucleic acid bases", and examples thereof include adenine, guanine, hypoxanthine, cytosine, thymine, uracil, etc., in which an amino group and/or a hydroxyl group is unprotected or protected.

In one aspect of the present invention, the amino-protective group is not particularly limited as long as it is used as a protective group for a nucleic acid, and specific examples thereof include benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl, and (dimethylamino)methylene. As the amino-protective group, benzoyl, acetyl, phenoxyacetyl, and 4-tert-butylphenoxyacetyl are preferable. Examples of the hydroxy-protective group include 2-cyanoethyl, 4-nitrophenethyl, phenylsulfonylethyl, methylsulfonylethyl, trimethylsilylethyl, phenyl optionally substituted with 1 to 5 electron-withdrawing groups at any substitutable positions, diphenylcarbamoyl, dimethylcarbamoyl, diethylcarbamoyl, methylphenylcarbamoyl, 1-pyrolidinylcarbamoyl, morpholinocarbamoyl, 4-(tert-butylcarboxy)benzyl, 4-[(dimethylamino)carboxy]benzyl, and 4-(phenylcarboxy)benzyl (see, for example, WO2009/064471A1). As the hydroxy-protective group, 2-cyanoethyl, 4-nitrophenethyl, and 4-(tert-butylcarboxy)benzyl are preferable. A protective group for the hydroxyl group at the 6-position of guanine is preferably 2-cyanoethyl.

In one aspect of the present invention, examples of the protected nucleic acid base include groups of the following formulae: 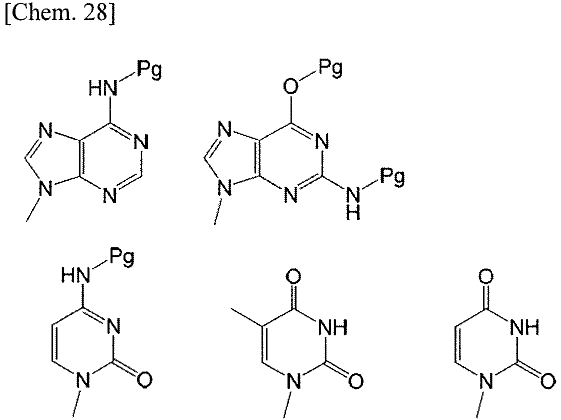 [wherein Pg represents a protective group].

In one aspect of the present invention, examples of protected nucleic acid bases include, but are not limited to, adenine having an amino group protected by benzoyl (A^{Bz}), cytosine having an amino group protected by benzoyl (C^{Bz}), guanine having a hydroxyl group protected by 2-cyanoethyl and an amino group protected by phenoxyacetyl (G^{CE}), etc.

In one aspect of the present invention, the solid-phase support is not particularly limited as long as it is a support that can be used for solid-phase reactions of nucleic acids, for example. However, for example, the solid-phase support is preferably a support that (i) is almost insoluble in reagents that can be used for the synthesis of morpholino nucleic acid oligomers (e.g., dichloromethane, acetonitrile, N-ethylmorpholine, N,N-diisopropylethylamine, N-methylimidazole, pyridine, acetic anhydride, 2,6-lutidine, 2,4-lutidine, trifluoroacetic acid), (ii) is chemically stable with respect to reagents that can be used for the synthesis of morpholino nucleic acid derivatives, (iii) can be chemically modified, (iv) can be charged with desired morpholino nucleic acid derivatives, (v) has strength sufficient to withstand a high pressure applied during treatment, and (vi) has a uniform particle size range and distribution.

In one aspect of the present invention, examples of the solid-phase support include swellable polystyrenes (e.g., Aminomethyl Polystyrene Resin cross-linked with 1% divinylbenzene (200 to 400 mesh) (2.4 to 3.0 mmol/g) (manufactured by Tokyo Chemical Industry Co., Ltd.), Aminomethylated Polystyrene Resin·HCl [divinylbenzene 1%, 100 to 200 mesh] (manufactured by PEPTIDE INSTITUTE, INC.), Aminomethyl Resin [Polystyrene 1% Divinylbenzene copolymer, 200 to 400 mesh] (manufactured by WATANABE CHEMICAL INDUSTRIES, LTD.)), non-swellable polystyrenes (e.g., Primer Support (manufactured by GE HealthCare)), PEG chain-attached polystyrenes (e.g., NH2-PEG resin (manufactured by WATANABE CHEMICAL INDUSTRIES, LTD.), TentaGel resin), controlled pore glass (CPG) (manufactured by, for example, CPG), oxalylized controlled pore glass (see, for example, Alul et al., Nucleic Acids Research, Vol. 19, 1527 (1991)), TentaGel support-amino polyethylene glycol-derivatized supports (see, for example, Wright et al., Tetrahedron Letters, Vol. 34, 3373 (1993)), and a copolymer of Poros-polystyrene/divinylbenzene.

In one aspect of the present invention, as the linker, any known linker used to link nucleic acids and morpholino nucleic acid derivatives can be used, and examples of the linker include short-chain alkylene, long-chain alkylene, amino-short-chain alkylene, amino-long-chain alkylene, diacyl short-chain alkylene (e.g., succinyl), diacyl long-chain alkylene, dialkylene sulfonyl, preferably 3-aminopropyl, succinyl, 2,2'-diethanol sulfonyl, and long-chain alkylamino (LCAA).

In one aspect of the present invention, L may be a group represented by formula (IV): 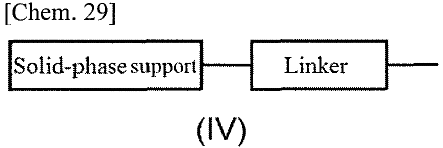 and may be a group represented by formula (IV-1): 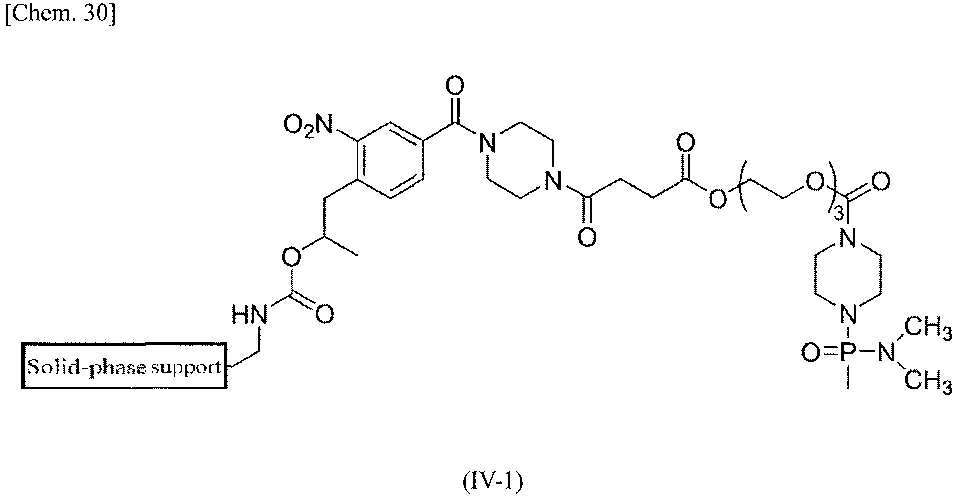 or a group represented by formula (IV-2):

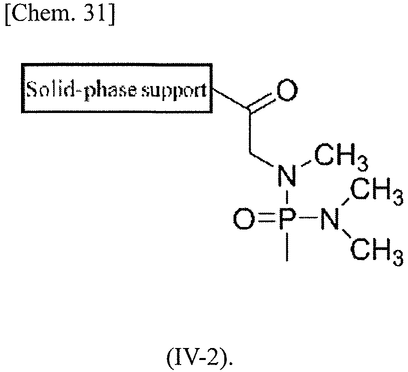

In one aspect of the present invention, a compound of formula (II): 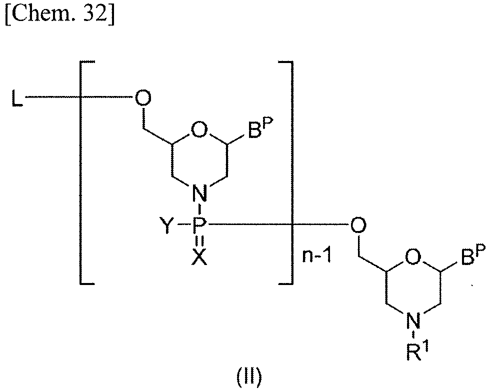 [wherein L, B^{P}, X, Y, R¹, and n are as described above] can be prepared by a method known in the art (see, for example, WO2012/043730).

### <Binding to solid-phase support and linker>

One aspect of the present invention may include a step of preparing a compound of formula (IIa) (hereinafter referred to as compound (IIa)): 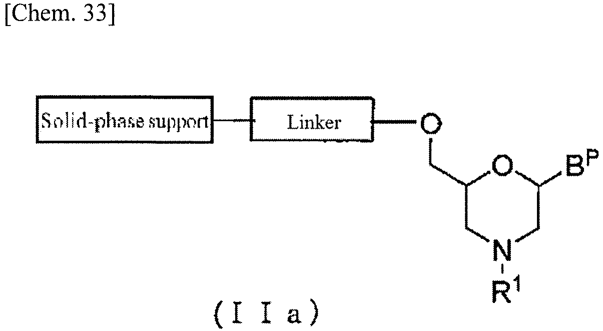 [wherein B^{P}, R¹, the linker, and the solid-phase support are as described above] that is the compound (II) in which n is 1 and L is the group represented by formula (IV).

One aspect of the present invention may include a step of preparing a compound of formula (VI) (hereinafter referred to as compound (VI)) by the action of an acylating agent on a compound of formula (V): 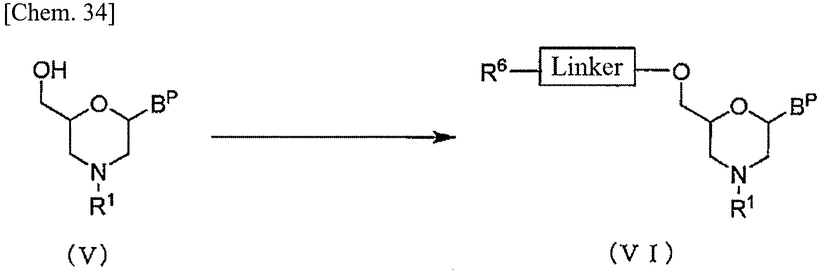 [wherein
B^{P} and R¹ are as defined above, and the linker is diacyl short-chain alkylene (e.g., succinyl), diacyl long-chain alkylene, short-chain alkylene, long-chain alkylene, or dialkylene sulfonyl, and
R⁶ is a hydroxyl group, a halogen, or amino].

This step can be carried out by a known linker introduction reaction using the compound (V) as a starting material.

One aspect of the present invention may include a step of preparing a compound of formula (VIa): 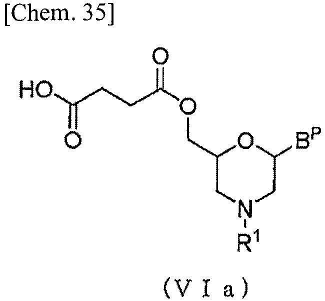 [wherein B^{P} and R¹ are as described above] by carrying out a method known as an esterification reaction using the compound (V) and succinic anhydride.

One aspect of the present invention may include a step of preparing the compound (IIa) by the action of a condensing agent or the like on the compound (VI) to react with the solid-phase support: 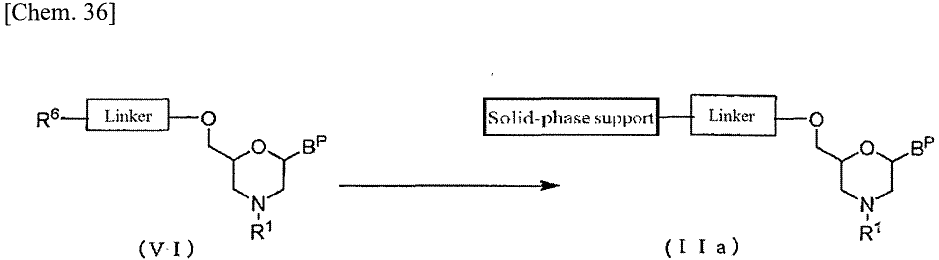 [wherein B^{P}, R⁶, R¹, the linker, and the solid-phase support are as described above].

This step can be carried out by a method known as a condensation reaction using the compound (VI) and the solid-phase support.

### <Raw materials>

In one aspect of the present invention, a compound of formula (VIII): 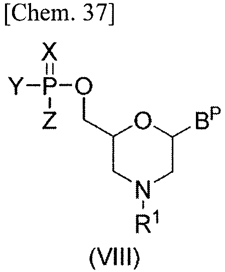 [wherein B^{P}, X, Y, Z, and R¹ are as described above] is commercially available or can be prepared and used by a method known in the art.

In one aspect of the present invention, examples of the compound of formula (VIII) include compounds listed in Table 1 below.

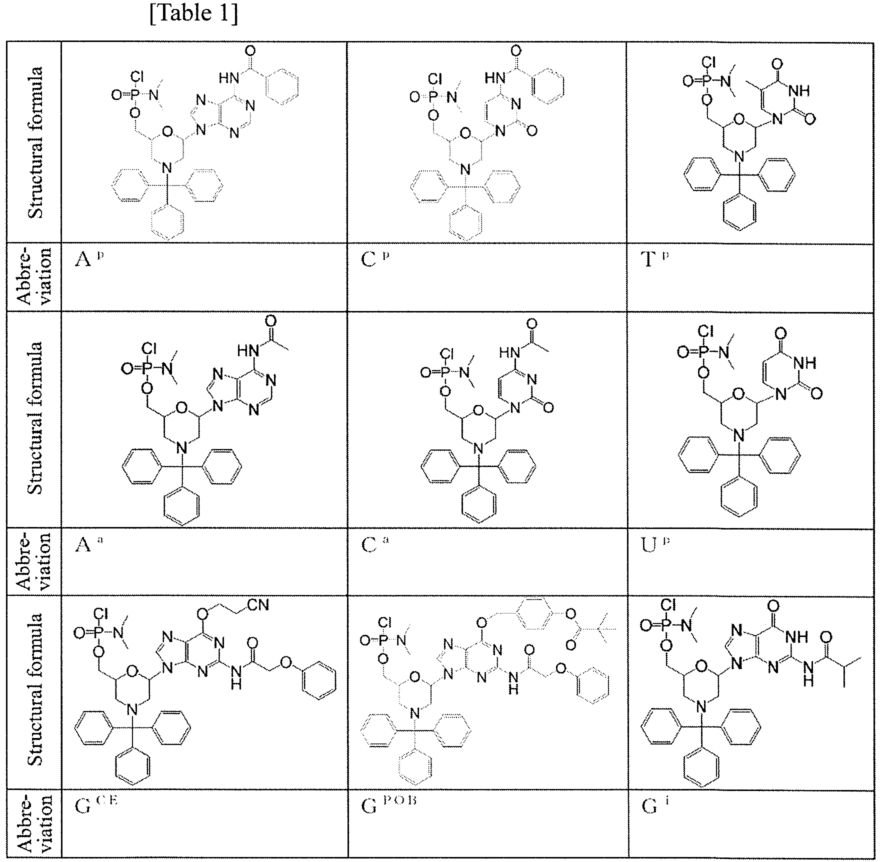

One aspect of the present invention is a preparation method for a morpholino nucleic acid oligomer, including, in the elongation reaction, at least one step selected from the group consisting of
<(1) Washing step before deprotection step>,
<(2) Deprotection step>,
<(3) Neutralization step>,
<(4) Washing step after neutralization step>,
<(5) Washing step before condensation>,
<(6) Condensation step>,
<(7) Washing step 1 after condensation step>, and
<(8) Washing step 2 after condensation step>.

Hereinafter, the steps (1) to (8) are described.

### <(1) Washing step before deprotection step>

This step includes (1) treating a compound of formula (II): 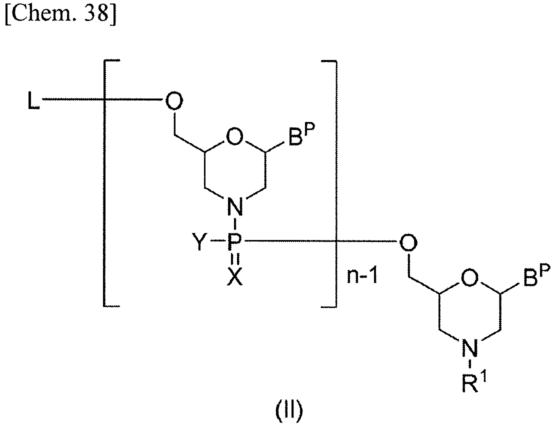
[wherein L, B^{P}, X, Y, R¹, and n are as described above]
with a solution containing an alcohol and/or a halogen solvent.

### <Solution in (1)>

In one embodiment, the solution in the step (1) is not particularly limited as long as it contains an alcohol and/or a halogen solvent. For example, at least one selected from the group consisting of 2,2,2-trifluoroethanol, difluoroethanol, chloroform, and dichloromethane can be used, and 2,2,2-trifluoroethanol and dichloromethane can be preferably used.

### <Solution in (1)>

In one embodiment, in the solution in the step (1), the alcohol can be used in a volume ratio of, for example, 0.01 to 100 times and preferably 0.1 to 2 times (v/v) with respect to the halogen solvent.

### <Amount of solution in (1)>

In one embodiment, the amount of the solution in the step (1) is not particularly limited, and is, for example, in the range of 1 g to 1000 g and preferably in the range of 2 g to 100 g per gram of the compound (II).

### <Treatment time in (1)>

In one embodiment, the time of the step (1) is not particularly limited, and is, for example, 0.1 minutes to 24 hours, preferably 1 minute to 5 hours, and further preferably 3 minutes to 1 hour.

### <Treatment temperature in (1)>

In one embodiment, the temperature in the step (1) is not particularly limited, and is, for example, 0°C to 40°C, preferably 10°C to 35°C, and further preferably 15°C to 30°C.

### <Number of times of step (1)>

In one embodiment, the number of times of the step (1) is not particularly limited, and is, for example, 1 to 10 and preferably 1 to 5.

### <(2) Deprotection step>

This step includes (2) treating the compound of formula (II): 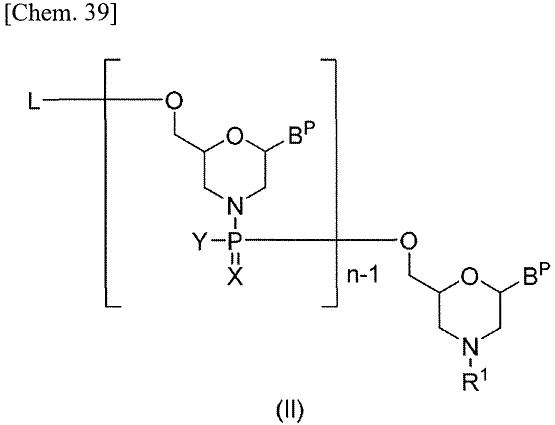
[wherein L, B^{P}, X, Y, R¹, and n are as described above]
with a solution containing an acid and a scavenger, to remove R¹ from the compound of formula (II) to obtain a compound of formula (III): 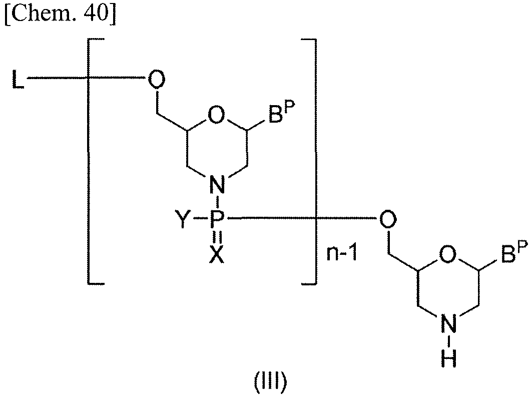
[wherein L, B^{P}, X, Y, and n are as described above].

### <Acid in (2)>

In one embodiment, examples of the acid include hydrochloric acid, trifluoroacetic acid, dichloroacetic acid, and trichloroacetic acid, and trifluoroacetic acid is preferable.

### <Amount of acid in (2)>

In one embodiment, the amount of the acid is, for example, in the range of 1 mole to 500 moles and preferably in the range of 2 moles to 100 moles per mole of the compound (II).

### <Concentration of acid in (2)>

In one embodiment, the acid can also be used after being diluted with an appropriate solvent to a concentration in the range of 0.1% to 30%.

### <Solvent for diluting acid in (2)>

In one embodiment, the solvent to be used is not particularly limited as long as it is not involved in the reaction, and examples of the solvent include dichloromethane, toluene, acetonitrile, alcohols (ethanol, isopropyl alcohol, 2,2,2-trifluoroethanol, etc.), water, and mixtures thereof.

### <Organic amine in (2)>

In one embodiment, an "organic amine" can be used together with the acid. While the organic amine is not particularly limited, examples of the organic amine include triethylamine, N,N-diisopropylethylamine, N-ethylmorpholine, pyridine, etc., and triethylamine is preferable.

### <Amount of organic amine in (2)>

In one embodiment, the amount of the organic amine is, for example, in the range of 0.01 moles to 10 moles and preferably in the range of 0.1 moles to 2 moles per mole of the acid.

### <Ratio of acid and organic amine in (2)>

In one embodiment, in the case of using a salt or mixture of the acid with the organic amine, the salt or mixture is, for example, a salt or mixture of trifluoroacetic acid with triethylamine, and is, for example, a mixture of 1.2 equivalents of trifluoroacetic acid and 1 equivalent of triethylamine.

### <Scavenger in (2)>

In one embodiment, the scavenger is, for example, at least one selected from the group consisting of alcohols (e.g., methanol, ethanol, and 2,2,2-trifluoroethanol), thiols (mercaptoethanol, dithiothreitol, mercaptosuccinic acid), organosilicon compounds (e.g., triisopropylsilane, triethylsilane, trimethylsilane), and aromatic compounds (e.g., 1-hydroxybenzotriazole, pyrrole, indole, anisole, and thioanisole), and organosilicon compounds are preferable.

### <Scavenger in (2)>

In one embodiment, the scavenger is, for example, at least one selected from the group consisting of methanol, ethanol, mercaptoethanol, 2,2,2-trifluoroethanol, triisopropylsilane, triethylsilane, trimethylsilane, 1-hydroxybenzotriazole, pyrrole, indole, anisole, and thioanisole, preferably one selected from the group consisting of triisopropylsilane and ethanol, and further preferably triisopropylsilane.

### <Amount of scavenger in (2)>

In one embodiment, the amount of the scavenger is, for example, in the range of 0.1 moles to 1000 moles, preferably in the range of 0.5 moles to 100 moles, and further preferably in the range of 1 mole to 20 moles per mole of the compound (II).

### <Reaction time in (2)>

In one embodiment, the reaction time is, for example, in the range of 0.1 minutes to 24 hours and preferably in the range of 1 minute to 3 hours.

### <Reaction temperature in (2)>

In one embodiment, the reaction temperature is, for example, preferably in the range of 0°C to 40°C, more preferably in the range of 10°C to 35°C, and further preferably in the range of 15°C to 30°C.

### <Number of times of step (2)>

In one embodiment, the number of times of the step (2) is not particularly limited, and is, for example, 1 to 10 and preferably 1 to 8.

### <Consecutive reactions of (1) washing before deprotection and (2) deprotection>

In one embodiment of the present invention, the compound of formula (II): 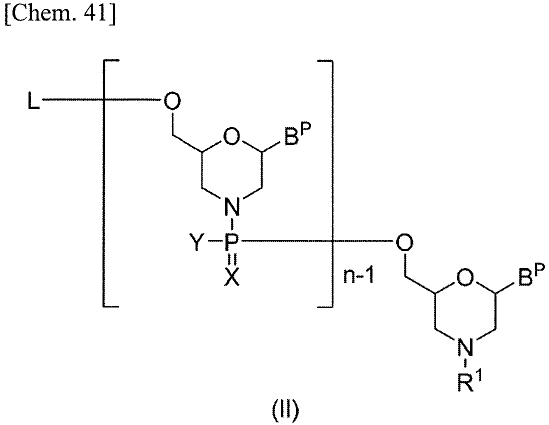
[wherein L, B^{P}, X, Y, R¹, and n are as described above]
   (1) can be treated with a solution containing an alcohol and/or a halogen solvent, and subsequently,
   (2) can be treated with a solution containing an acid and a scavenger, to remove R¹ from the compound of formula (II) to obtain the compound of formula (III): 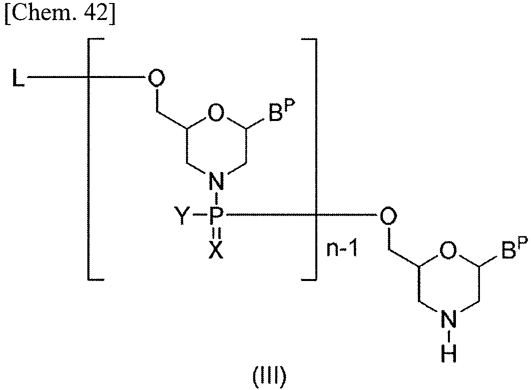
[wherein L, B^{P}, X, Y, and n are as described above].

The steps (1) and (2) are as described above, and can be combined as appropriate.

### <(3) Neutralization step>

This step includes (3) treating the compound of formula (III): 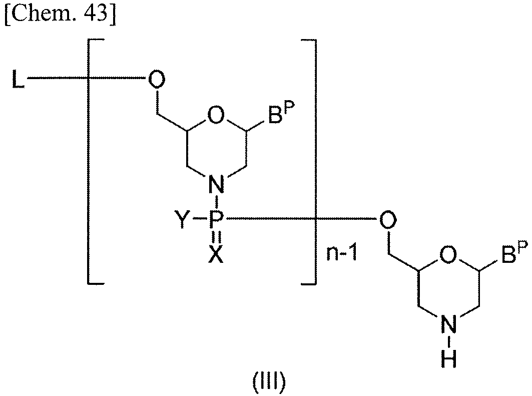
[wherein L, B^{P}, X, Y, and n are as described above]
with a solution containing a base and a solvent (alcohol and halogen solvent).

### <Base in (3)>

In one embodiment, while the base is not particularly limited as long as it does not affect the protecting group, examples of the base include triethylamine, N,N-diisopropylethylamine, pyridine, etc., and N,N-diisopropylethylamine is preferable.

### <Amount of base in (3)>

In one embodiment, as for the amount of the base, the base can also be used after being diluted with an appropriate solvent to a concentration in the range of 0.1% (v/v) to 30% (v/v).

### <Amount of solution in (3)>

In one embodiment, the amount of the solution in the step (3) is not particularly limited, and is, for example, in the range of 1 g to 1000 g and preferably in the range of 2 g to 100 g per gram of the compound (III).

### <Solvent in (3)>

In one embodiment, the solvent is at least one selected from the group consisting of alcohols, halogen solvents, polar solvents, ether-based solvents, and mixtures thereof, and mixtures of alcohols and halogen solvents are preferable.

In one embodiment, the alcohols are ethanol, isopropyl alcohol, 2,2,2-trifluoroethanol, etc., and isopropyl alcohol is preferable.

In one embodiment, the halogen solvents are chloroform, dichloromethane, tetrachloroethane, tetrachloroethylene, etc., and dichloromethane is preferable.

In one embodiment, the polar solvents are acetonitrile, dimethylsulfoxide, etc.

In one embodiment, the ether-based solvents are tetrahydrofuran, cyclopentyl methyl ether, etc.

### <Reaction time in (3)>

In one embodiment, the reaction time is different depending on the type of the base to be used and the reaction temperature, and is, for example, in the range of 0.1 minutes to 24 hours and preferably in the range of 1 minute to 5 hours.

### <Reaction temperature in (3)>

In one embodiment, the reaction temperature is, for example, preferably in the range of 0°C to 40°C, more preferably in the range of 1°C to 35°C, and further preferably in the range of 3°C to 20°C.

### <Number of times of step (3)>

In one embodiment, the number of times of the step (3) is not particularly limited, and is, for example, 1 to 10 and preferably 1 to 5.

### <Consecutive reactions of (2) deprotection and (3) neutralization>

In one embodiment of the present invention,
(2) the compound of formula (II): 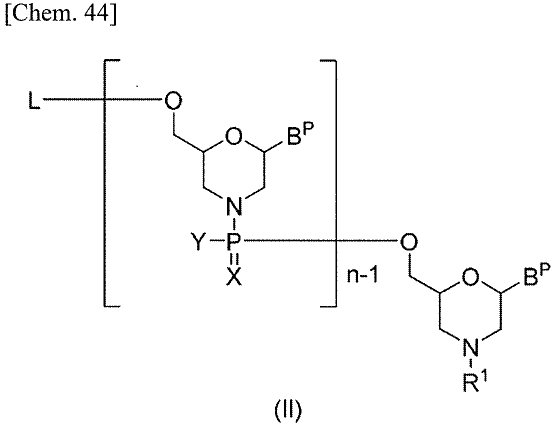
[wherein L, B^{P}, X, Y, R¹, and n are as described above]
can be treated with a solution containing an acid and a scavenger, to remove R¹ from the compound of formula (II) to obtain the compound of formula (III): 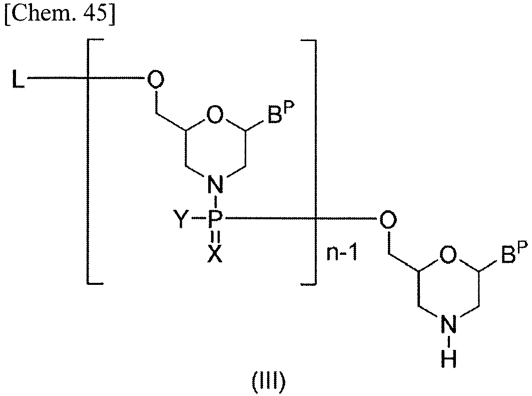
[wherein L, B^{P}, X, Y, and n are as described above], and subsequently,
(3) the compound of formula (III): 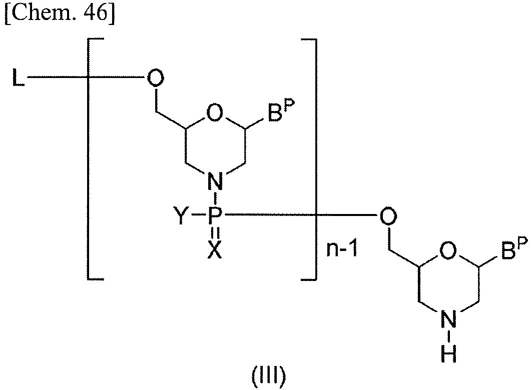
[wherein L, B^{P}, X, Y, and n are as described above]
can be treated with a solution containing a base and a solvent (alcohol and halogen solvent).

The steps (2) and (3) are as described above, and can be combined as appropriate.

### <(4) Washing step after neutralization step>

This step includes (4) treating the compound of formula (III): 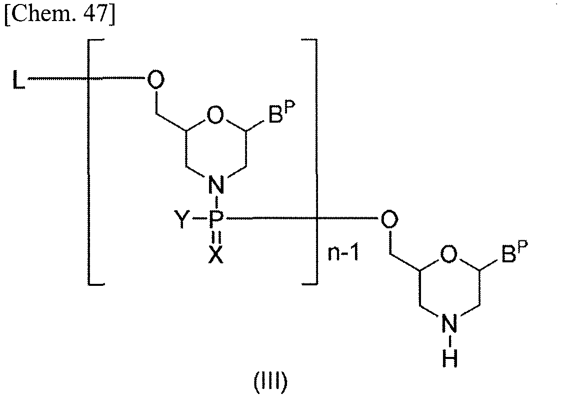
[wherein L, B^{P}, X, Y, and n are as described above]
with a solution containing a halogen solvent.

### <Solution in (4)>

In one embodiment, the solution in the step (4) is not particularly limited as long as it contains a halogen solvent. For example, at least one selected from the group consisting of chloroform, dichloromethane, tetrachloroethane, and tetrachloroethylene can be used, and dichloromethane is preferable.

### <Amount of solution in (4)>

In one embodiment, the amount of the solution in the step (4) is not particularly limited, and is, for example, in the range of 1 g to 1000 g and preferably in the range of 2 g to 100 g per gram of the compound (III).

### <Treatment time in (4)>

In one embodiment, the time of the step (4) is not particularly limited, and is, for example, 0.1 minutes to 2 hours, preferably 1 minute to 60 minutes, and further preferably 3 minutes to 30 minutes.

### <Treatment temperature in (4)>

In one embodiment, the temperature in the step (4) is not particularly limited, and is, for example, 0°C to 35°C, preferably 10°C to 30°C, and further preferably 15°C to 25°C.

### <Number of times of step (4)>

In one embodiment, the number of times of the step (4) is not particularly limited, and is, for example, 1 to 10 and preferably 1 to 5.

### <Consecutive reactions of (2) deprotection, (3) neutralization, and (4) washing after neutralization>

In one embodiment of the present invention, the compound of formula (II): 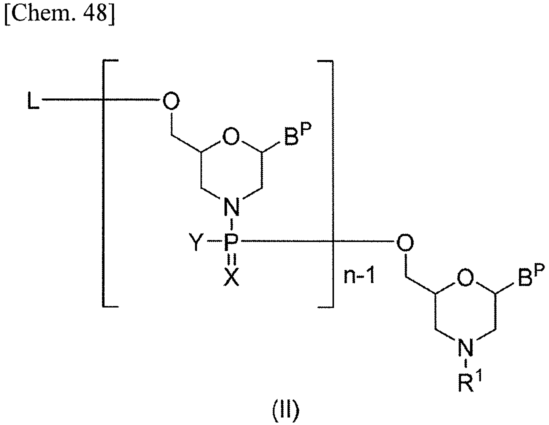
[wherein L, B^{P}, X, Y, R¹, and n are as described above]
(2) can be treated with a solution containing an acid and a scavenger, to remove R¹ from the compound of formula (II) to obtain the compound of formula (III): 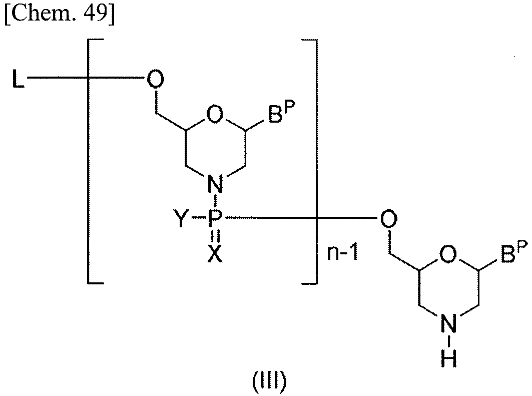
[wherein L, B^{P}, X, Y and n are as described above], and subsequently, the compound of formula (III): 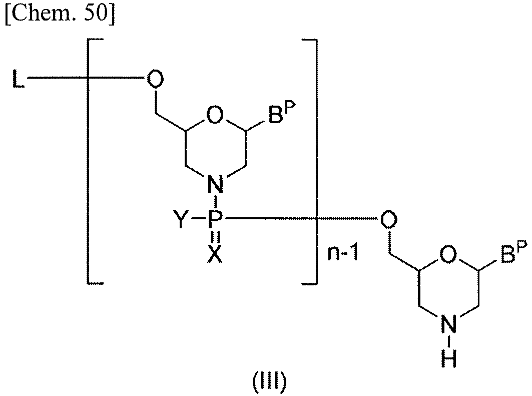
[wherein L, B^{P}, X, Y and n are as described above]
(3) can be treated with a solution containing a base and a solvent (alcohol and halogen solvent), and further
(4) can be treated with a solution containing a halogen solvent.

The steps (2), (3), and (4) are as described above, and can be combined as appropriate.

### <(5) Washing step before condensation>

This step includes (5) treating the compound of formula (III): 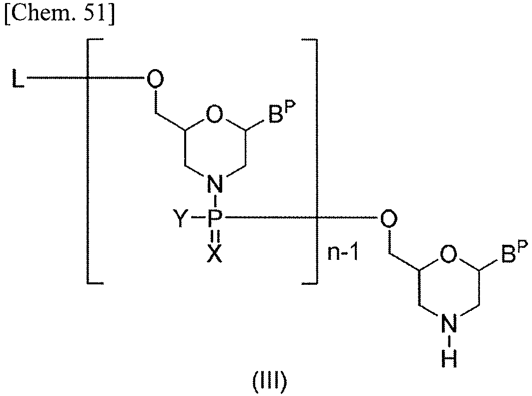
[wherein L, B^{P}, X, Y, and n are as described above]
with a solution containing an organic amine and an aprotic polar solvent.

### <Organic amine in (5)>

In one embodiment, the organic amine is, for example, at least one selected from the group consisting ofN,N-diisopropylethylamine, triethylamine, and N-ethylmorpholine, and N-ethylmorpholine is preferable.

### <Amount of organic amine in (5)>

In one embodiment, the amount of the organic amine is, for example, in the range of 1 mole to 1000 moles and preferably in the range of 1 mole to 100 moles per mole of the compound of formula (III).

### <Aprotic polar solvent in (5)>

In one embodiment, the aprotic polar solvent is, for example, at least one selected from the group consisting of 1,3-dimethyl-2-imidazolidinone, N-methylpyrrolidone, N,N-dimethylformamide, tetrahydrofuran, and mixtures thereof, and 1,3-dimethyl-2-imidazolidinone is preferable.

### <Amount of aprotic polar solvent in (5)>

In one embodiment, the amount of the aprotic polar solvent is, for example, in the range of 1 to 1000 times (v/v) and preferably in the range of 3 to 100 times the volume of the organic amine.

### <Amount of solution in (5)>

In one embodiment, the amount of the solution in the step (5) is not particularly limited, and is, for example, in the range of 0.5 g to 100 g and preferably in the range of 1 g to 50 g per gram of the compound (III).

### <Treatment time in (5)>

In one embodiment, the treatment time is different depending on the type of the organic amine to be used and the treatment temperature, and is usually in the range of 0.1 minutes to 2 hours and preferably in the range of 1 minute to 60 minutes.

### <Treatment temperature in (5)>

In one embodiment, the treatment temperature is, for example, preferably in the range of 0°C to 100°C and more preferably in the range of 10°C to 50°C.

### <Number of times of step (5)>

In one embodiment, the number of times of the step (5) is not particularly limited, and is, for example, 1 to 10 and preferably 1 to 5.

### <(6) Condensation step>

This step includes (6) treating the compound of formula (VIII): 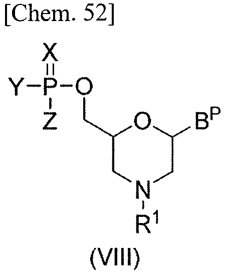
[wherein B^{P}, X, Y, Z, and R¹ are as described above]
with a solution containing an organic amine and an aprotic polar solvent, for example, dissolving the compound of formula (VIII) in a mixed solution containing an organic amine and an aprotic polar solvent.

### <Organic amine in (6)>

In one embodiment, the organic amine is, for example, at least one selected from the group consisting of N,N-diisopropylethylamine, triethylamine, and N-ethylmorpholine, and N-ethylmorpholine is preferable.

### <Amount of organic amine in (6)>

In one embodiment, the amount of the organic amine is, for example, in the range of 0.1 moles to 1000 moles and preferably in the range of 1 mole to 100 moles per mole of the compound of formula (VIII).

### <Aprotic polar solvent in (6)>

In one embodiment, the aprotic polar solvent is, for example, at least one selected from the group consisting of 1,3-dimethyl-2-imidazolidinone, N-methylpyrrolidone, N,N-dimethylformamide, tetrahydrofuran, and mixtures thereof, and 1,3-dimethyl-2-imidazolidinone is preferable.

### <Amount of aprotic polar solvent in (6)>

In one embodiment, the amount of the aprotic polar solvent is, for example, in the range of 1 to 1000 times (v/v) and preferably in the range of 3 to 100 times the volume of the organic amine.

### <Treatment time in (6)>

In one embodiment, the treatment time is different depending on the type of the organic amine to be used and the treatment temperature, and is usually in the range of 0.1 minutes to 48 hours and preferably in the range of 1 minute to 24 hours.

### <Treatment temperature in (6)>

In one embodiment, the treatment temperature is, for example, preferably in the range of 0°C to 50°C and more preferably in the range of 10°C to 40°C.

This step further includes reacting the compound of formula (III): 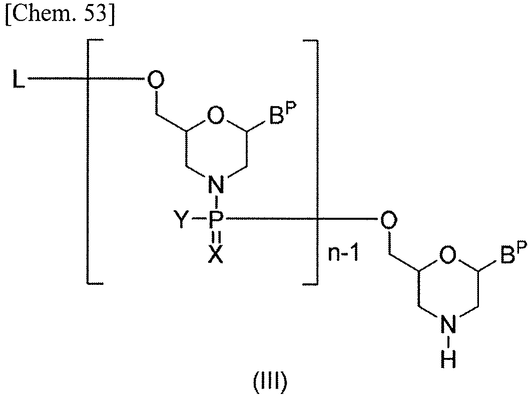
[wherein L, B^{P}, X, Y, and n are as described above]
in the presence of an organic amine with the compound of formula (VIII): 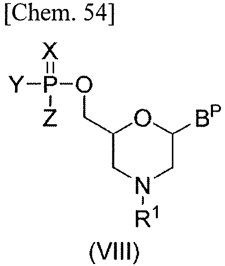
[wherein X, Y, Z, B^{P}, and R¹ are as described above]
to obtain a compound of formula (II'): 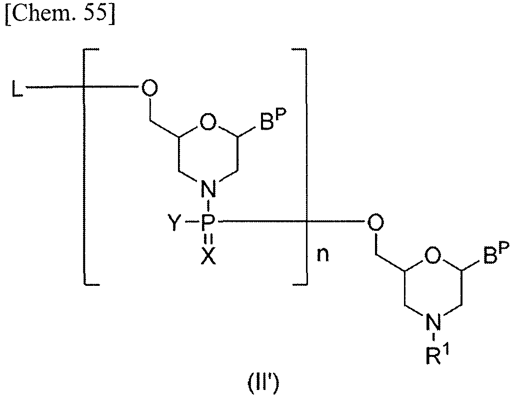
[wherein L, B^{P}, X, Y, R¹, and n are as described above].

In one embodiment, the compound of formula (VIII) is, for example, in the range of 1 mole to 10 moles and preferably in the range of 1 mole to 5 moles per mole of the compound of formula (III).

In one embodiment, the organic amine is, for example, at least one selected from the group consisting of N,N-diisopropylethylamine, triethylamine, and N-ethylmorpholine, and N-ethylmorpholine is preferable.

In one embodiment, the amount of the organic amine is, for example, in the range of 0.1 moles to 1000 moles and preferably in the range of 1 mole to 100 moles per mole of the compound of formula (VIII).

In one embodiment, the reaction time is different depending on the type of the organic amine to be used and the reaction temperature, and is usually in the range of 10 minutes to 48 hours and preferably in the range of 30 minutes to 24 hours.

In one embodiment, the reaction temperature is, for example, preferably in the range of 0°C to 100°C and more preferably in the range of 10°C to 50°C.

### <Number of times of step (6)>

In one embodiment, the number of times of the step (6) is not particularly limited, and is, for example, 1 to 3 and preferably 1.

### <Consecutive reactions of washing, pretreatment, and condensation>

In one embodiment of the present invention,
(5) the compound of formula (III): 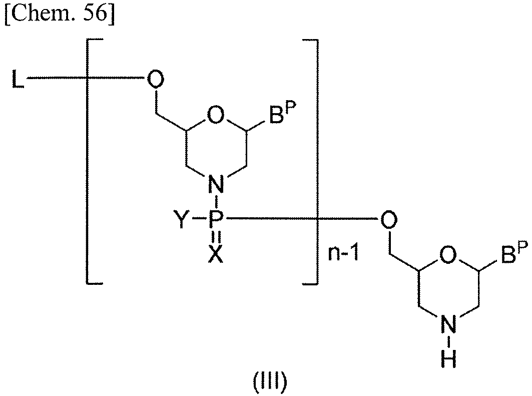
[wherein L, B^{P}, X, Y, and n are as described above]
can be treated with a solution containing an organic amine and an aprotic polar solvent, and
(6) the compound of formula (VIII): 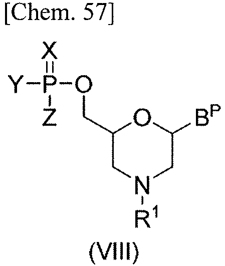
[wherein B^{P}, X, Y, Z, and R¹ are as described above]
can be treated with a solution containing an organic amine and an aprotic polar solvent, and then the compound of formula (III): 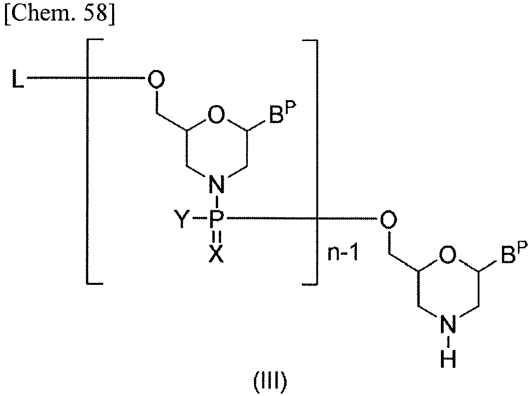
[wherein L, B^{P}, X, Y, and n are as described above]
can be reacted in the presence of an organic amine with the compound of formula (VIII): 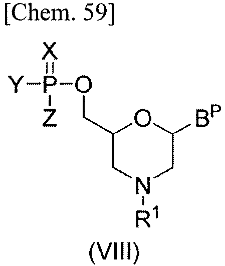
[wherein X, Y, Z, B^{P}, and R¹ are as described above]
to obtain the compound of formula (II'): 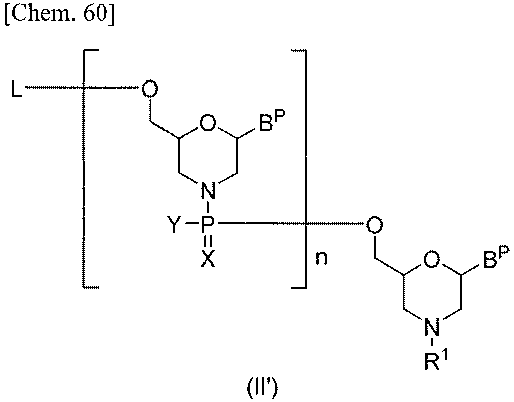
[wherein L, B^{P}, X, Y, R¹, and n are as described above].

The steps (5) and (6) are as described above, and can be combined as appropriate.

### <(7) Washing step 1 after condensation step>

This step includes (7) treating a compound of formula (II'): 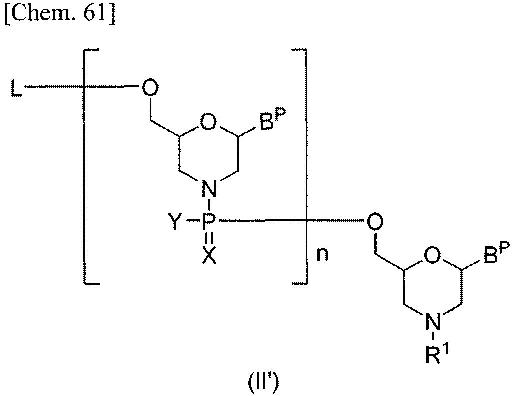
[wherein L, B^{P}, X, Y, R¹, and n are as described above]
with a solution containing a halogen solvent.

### <Solution in (7)>

In one embodiment, the washing agent in the step (7) is not particularly limited as long as it contains a halogen solvent. For example, at least one selected from the group consisting of chloroform, dichloromethane, tetrachloroethane, and tetrachloroethylene can be used, and dichloromethane is preferable.

### <Amount of solution in (7)>

In one embodiment, the amount of the washing agent in the step (7) is not particularly limited, and is, for example, in the range of 1 g to 1000 g and preferably in the range of 2 g to 100 g per gram of the compound (II').

### <Treatment time in (7)>

In one embodiment, the time of the step (7) is not particularly limited, and is, for example, 0.1 minutes to 24 hours, preferably 1 minute to 5 hours, and further preferably 3 minutes to 1 hour.

### <Treatment temperature in (7)>

In one embodiment, the temperature in the step (7) is not particularly limited, and is, for example, 0°C to 40°C, preferably 10°C to 35°C, and further preferably 15°C to 30°C.

### <Consecutive reactions of condensation and washing after condensation>

In one embodiment of the present invention, (6) the compound of formula (III): 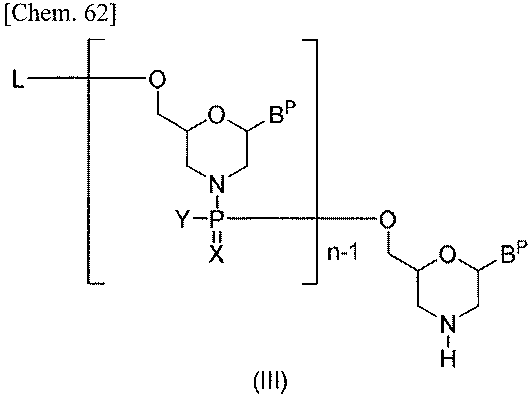
[wherein L, B^{P}, X, Y, and n are as described above]
can be reacted in the presence of an organic amine with the compound of formula (VIII): 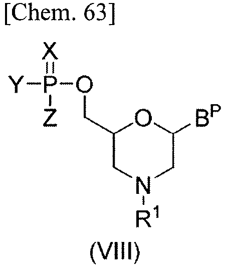
[wherein X, Y, Z, B^{P}, and R¹ are as described above]
to obtain the compound of formula (II'): 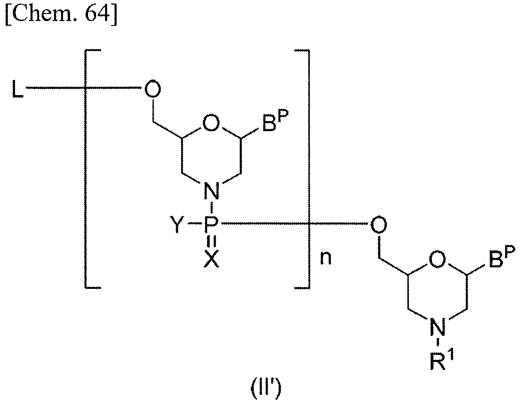
[wherein L, B^{P}, X, Y, R¹, and n are as described above], and subsequently,
(7) the compound of formula (II') can be treated with a solution containing a halogen solvent.

The steps (6) and (7) are as described above, and can be combined as appropriate.

### <(8) Washing step 2 after condensation step>

This step includes (8) treating the compound of formula (II'): 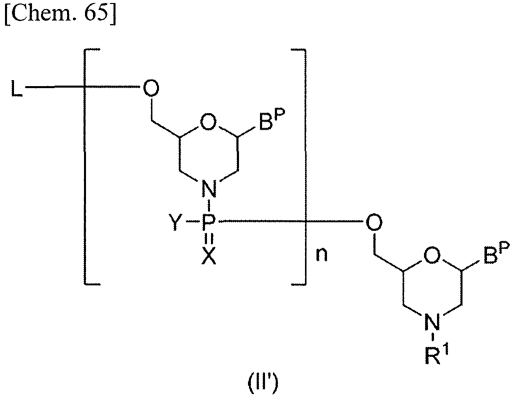
[wherein L, B^{P}, X, Y, R¹, and n are as described above]
with a solution containing an alcohol and/or a halogen solvent.

### <Solution in (8)>

In one embodiment, the solution in the step (8) is not particularly limited as long as it contains an alcohol and/or a halogen solvent. For example, at least one selected from the group consisting of 2,2,2-trifluoroethanol, difluoroethanol, chloroform, and dichloromethane can be used, and 2,2,2-trifluoroethanol and dichloromethane can be preferably used.

### <Solution in (8)>

In one embodiment, in the solution in the step (8), the alcohol can be used in a ratio of, for example, 0.01 to 100 times and preferably 0.1 to 2 times (v/v) with respect to the halogen solvent.

### <Amount of solution in (8)>

In one embodiment, the amount of the solution in the step (8) is not particularly limited, and is, for example, in the range of 1 g to 1000 g and preferably in the range of 2 g to 100 g per gram of the compound (II').

### <Treatment time in (8)>

In one embodiment, the time of the step (8) is not particularly limited, and is, for example, 0.1 minutes to 24 hours, preferably 1 minute to 5 hours, and further preferably 3 minutes to 1 hour.

### <Treatment temperature in (8)>

In one embodiment, the temperature in the step (8) is not particularly limited, and is, for example, 0°C to 40°C, preferably 10°C to 35°C, and further preferably 15°C to 30°C.

### <Number of times of step (8)>

In one embodiment, the number of times of the step (8) is not particularly limited, and is, for example, 1 to 10 and preferably 1 to 5.

### <Consecutive reactions of condensation and washing after condensation>

In one embodiment of the present invention, (6) the compound of formula (III): 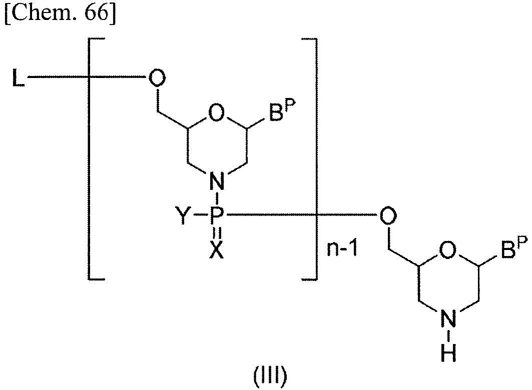
[wherein L, B^{P}, X, Y, and n are as described above]
and the compound of formula (VIII): 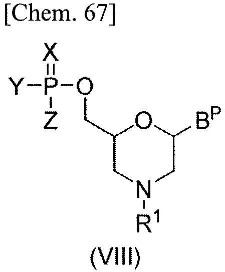
[wherein X, Y, Z, B^{P}, and R¹ are as described above]
can be reacted to obtain the compound of formula (II'): 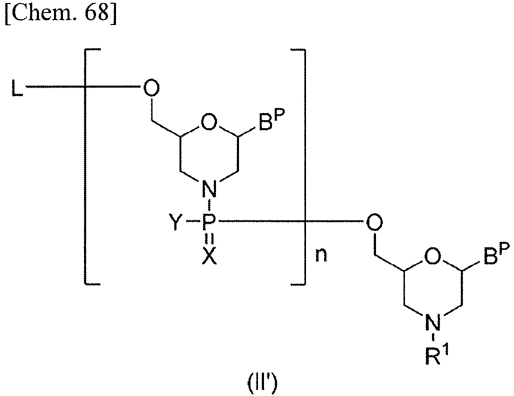
[wherein L, B^{P}, X, Y, R¹, and n are as described above], and subsequently, the compound of formula (II')
(7) can be treated with a solution containing a halogen solvent, and further
(8) can be treated with a solution containing an alcohol and/or a halogen solvent.

The steps (6), (7), and (8) are as described above, and can be combined as appropriate.

One aspect of the present invention is a preparation method for a morpholino nucleic acid oligomer, including, subsequent to the elongation reaction: 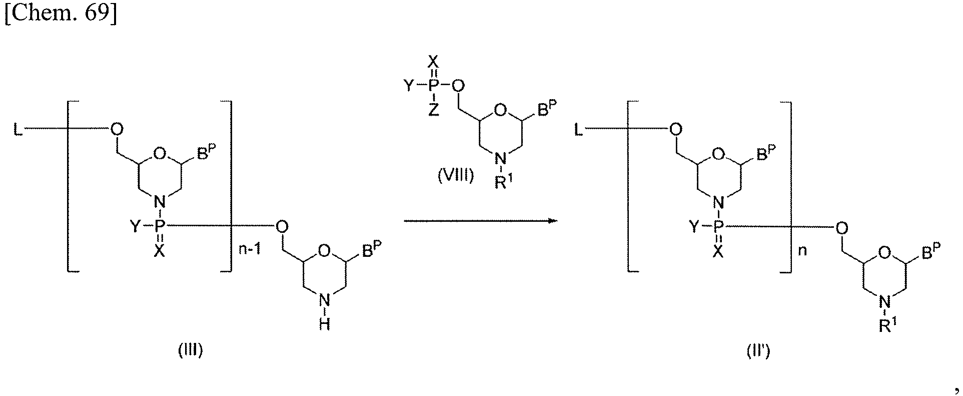 [wherein B^{P}, R¹, L, n, X, Y and Z are as described above], at least one step selected from among
<(A) Removal step of protective group>,
<(B) Removal step of protective group of morpholino nitrogen>, and
<(C) Purification step>.

Hereinafter, the steps (A) to (C) are described.

### <(A-1) Removal step of protective group>

In one embodiment, this step includes a step of obtaining a compound of formula (I") from the compound of formula (II"): 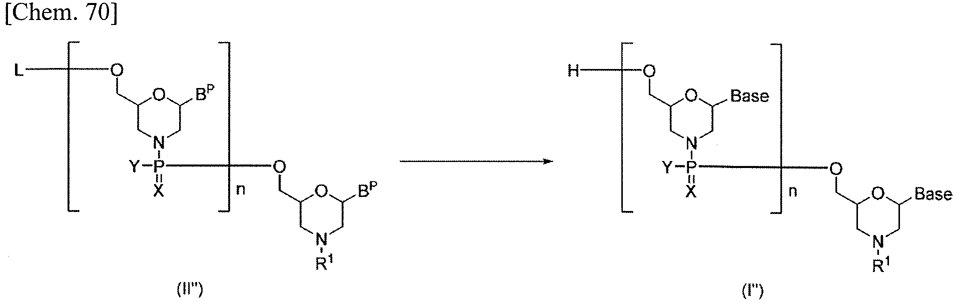 [wherein Base, B^{P}, R¹, L, n, X, and Y are as described above].

### <(A-2) Removal step of protective group>

In one embodiment, this step includes a step of obtaining the compound of formula (I) from a compound of formula (II‴): 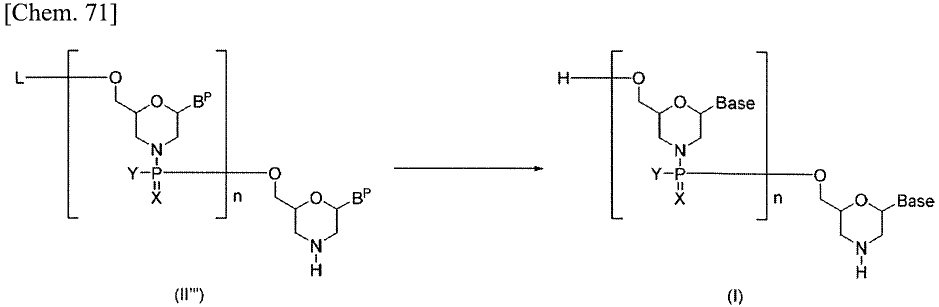 [wherein Base, B^{P}, L, n, X, and Y are as described above].

### <(A-3) Removal step of protective group>

In one embodiment, this step includes a step of obtaining a compound of formula (I"") from the compound of formula (II"): 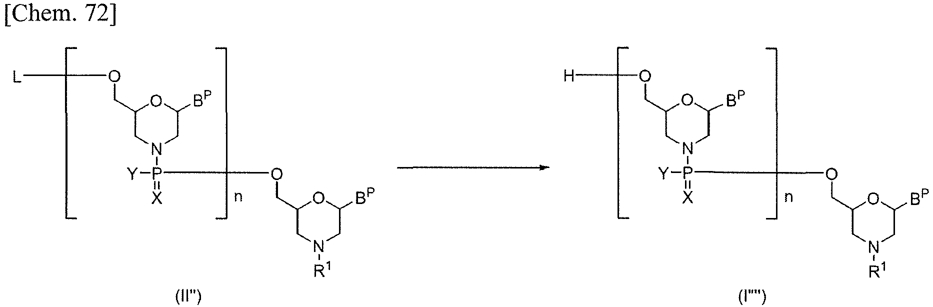 [wherein B^{P}, R¹, L, n, X, and Y are as described above].

### <(A-4) Removal step of protective group>

In one embodiment, this step includes a step of obtaining a compound of formula (I‴ʺ) from the compound of formula (II‴): 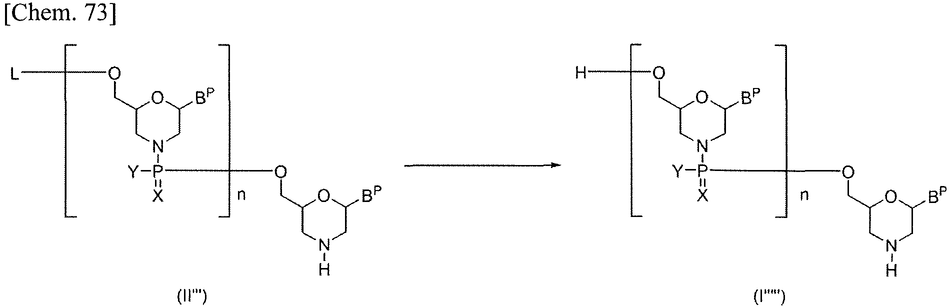 [wherein B^{P}, L, n, X, and Y are as described above].

In one embodiment,
L in the compound of formula (II") and the protective group of the optionally protected nucleic acid base in the compound of formula (II"); and
L in the compound of formula (II‴) and the protective group of the optionally protected nucleic acid base in the compound of formula (II‴),
can be subjected to a deprotection treatment according to the types or properties of the solid-phase support, the linker, and the protective group, to prepare the target compound. All the protective groups of the compound can be removed, for example, according to the deprotection method described in "Green's PROTECTIVE GROUPS in ORGANIC SYNTHESIS, 4th Edition, 2006". If necessary, the target compound can be subjected to further chemical structural transformation.

In one embodiment,
L in the compound of formula (II"); and
L in the compound of formula (II‴),
can be subjected to a deprotection treatment according to the types or properties of the solid-phase support and the linker, to prepare the target compound.

Furthermore, B^{P} and R¹ in the compound of formula (I""); and
B^{P} in the compound of formula (I‴ʺ)
can be subjected to a deprotection treatment according to the type or properties of the protective group, to prepare the target compound.

All the protective groups of the compound can be removed, for example, according to the method described in the present specification or the deprotection method described in "Green's PROTECTIVE GROUPS in ORGANIC SYNTHESIS, 4th Edition, 2006". If necessary, the target compound can be subjected to further chemical structural transformation.

In one embodiment,
L in the compound of formula (II") and the protective group of the optionally protected nucleic acid base in the compound of formula (II"); and
L in the compound of formula (II'") and the protective group of the optionally protected nucleic acid base in the compound of formula (II‴)
can be removed by performing a treatment, for example, with (1) ammonia water, (2) a mixed solution of ammonia water/ethanol, or (3) a mixed solution of methylamine-methanol solution/water, preferably with (2) a mixed solution of ammonia water/ethanol.

In one embodiment, the amount of (1) ammonia water, (2) the mixed solution of ammonia water/ethanol, or (3) the mixed solution of methylamine-methanol solution/water is, for example, in the range of 1 g to 1000 g and preferably in the range of 3 g to 100 g per gram of the compound of formula (II") or the compound of formula (II‴).

In one embodiment, the reaction time is different depending on the reaction temperature, etc., and is in the range of 10 minutes to 120 hours, preferably in the range of 30 minutes to 72 hours, and more preferably in the range of 5 hours to 48 hours.

In one embodiment, the reaction temperature is, for example, in the range of 5°C to 100°C, preferably in the range of 10°C to 70°C, and more preferably in the range of 15°C to 50°C.

### <(B-1) Removal step of protective group of morpholino nitrogen>

In one embodiment, this step includes a step of removing R¹ from the compound of formula (II') to obtain the compound of formula (II‴): 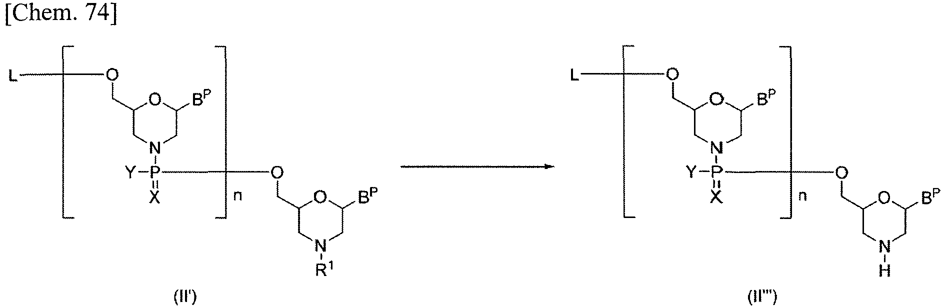 [wherein B^{P}, L, n, R¹, X, and Y are as described above].

### <(B-2) Removal step of protective group of morpholino nitrogen>

In one embodiment, this step includes a step of removing R¹ from the compound of formula (I') to obtain the compound of formula (I): 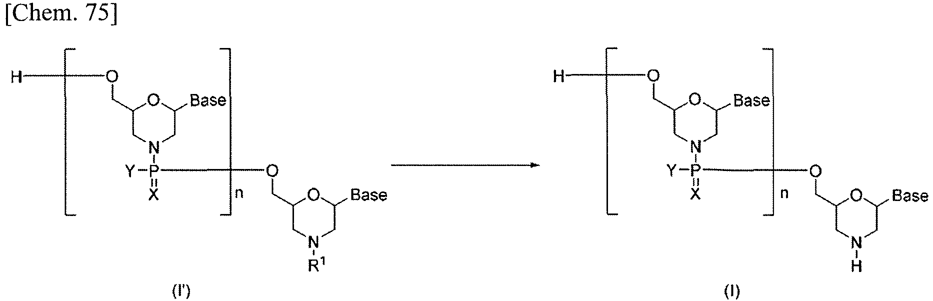 [wherein Base, X, Y, n, and R¹ are as described above].

In one embodiment, the conditions used in the above steps (1) to (4) can be applied to:
the step of removing R¹ from the compound of formula (II') to obtain the compound of formula (II‴); and
the step of removing R¹ from the compound of formula (I') to obtain the compound of formula (I).

### <(C) Purification step>

In one embodiment, this step includes isolating the morpholino nucleic acid oligomer (1) from a reaction mixture, using usual means of separation and purification, for example, using means such as extraction, concentration, neutralization, filtration, centrifugation, precipitation, recrystallization, C₁ to C₁₈ reverse phase column chromatography, cation exchange column chromatography, anion exchange column chromatography, gel filtration column chromatography, high performance liquid chromatography, dialysis, and ultra-filtration, alone or in combination (see, for example, WO1991/09033A1).

In one embodiment, in the case of purifying the target compound using reverse phase chromatography, for example, a mixed solution containing 20 mM triethylamine/acetate buffer and acetonitrile can be used as an elution solvent.

In one embodiment, in the case of purifying the target compound using ion exchange chromatography, for example, a mixed solution containing a 1 M solution of NaCl and a 10 mM aqueous solution of sodium hydroxide, or a 0.3 M NaCl solution in 50 mM phosphate buffer can be used.

In one aspect of the present invention, the preparation method of the present invention includes <(2) Deprotection step>.

In one aspect of the present invention, the preparation method of the present invention includes <(2) Deprotection step> and <(3) Neutralization step>.

In one aspect of the present invention, the preparation method of the present invention includes <(2) Deprotection step>, <(3) Neutralization step>, and <(4) Washing step after neutralization step>.

In one aspect of the present invention, the preparation method of the present invention includes <(5) Washing step before condensation> and <(6) Condensation step>.

In one aspect of the present invention, the preparation method of the present invention includes <(5) Washing step before condensation>, <(6) Condensation step>, and <(7) Washing step 1 after condensation step> and/or <(8) Washing step 2 after condensation step>.

In one aspect of the present invention, the preparation method of the present invention includes <(2) Deprotection step>, <(5) Washing step before condensation>, and <(6) Condensation step>.

In one aspect of the present invention, the preparation method of the present invention includes <(2) Deprotection step>, <(3) Neutralization step>, <(5) Washing step before condensation>, and <(6) Condensation step>.

In one aspect of the present invention, the preparation method of the present invention includes
<(1) Washing step before deprotection step>,
<(2) Deprotection step>,
<(3) Neutralization step>,
<(4) Washing step after neutralization step>,
<(5) Washing step before condensation>,
<(6) Condensation step>,
<(7) Washing step 1 after condensation step>, and
<(8) Washing step 2 after condensation step>.

In one aspect of the present invention, the preparation method of the present invention does not include <(5) Washing step before condensation>.

In one aspect of the present invention, the preparation method of the present invention includes
<(1) Washing step before deprotection step>,
<(2) Deprotection step>,
<(3) Neutralization step>,
<(4) Washing step after neutralization step>,
<(6) Condensation step>,
<(7) Washing step 1 after condensation step>, and
<(8) Washing step 2 after condensation step>.

In one aspect of the present invention, the preparation method of the present invention does not include <Capping step>, as the case may be.

With the preparation method for a morpholino nucleic acid oligomer of the present invention, it is possible to obtain the morpholino nucleic acid oligomer of formula (I): 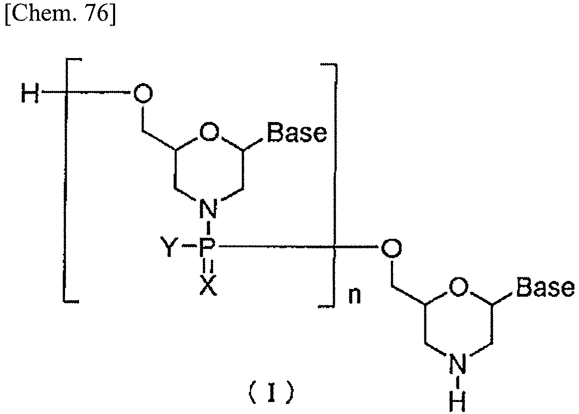 [wherein Base, X, Y, and n are as described above], for example, a morpholino nucleic acid oligomer of formula (I-1): 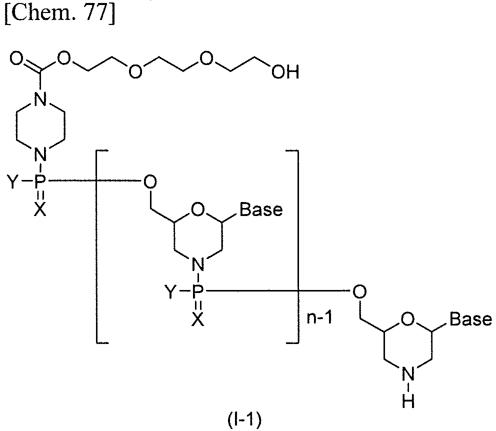 [wherein Base, X, Y, and n are as described above], or a morpholino nucleic acid oligomer of formula (I-2): 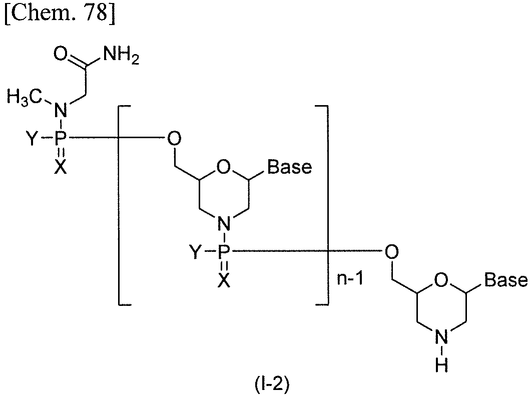 [wherein Base, X, Y, and n are as described above].

In one aspect of the present invention, the morpholino nucleic acid oligomer of formula (I): 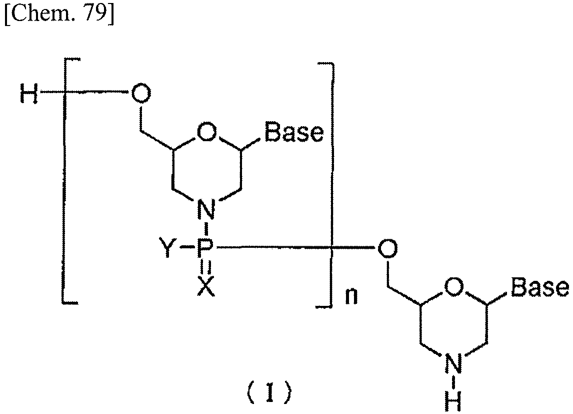 [wherein Base, X, Y, and n are as described above] is, for example, a phosphorodiamidate morpholino oligomer (PMO) that is described in international publications (WO2012/029986, WO2013/100190, WO2015/137409, WO2015/194520, WO2017/047707, WO2021/132591, WO2021/172498, etc.) or can be recognized by those skilled in the art based on the description of these international publications, and is preferably a phosphorodiamidate morpholino oligomer (PMO) capable of skipping at least one selected from the group consisting of exons 51, 53, 45, 55, 44, and 50 of the human dystrophin gene, and one specific example thereof is Viltepso (see WO2012/029986; CAS Registration Number: 2055732-84-6).

In one aspect of the present invention, the morpholino nucleic acid oligomer of formula (I-1): 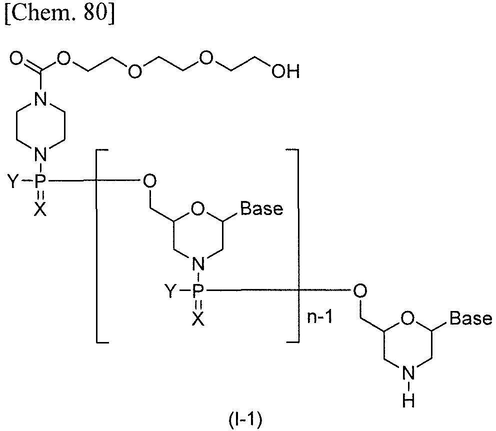 [wherein Base, X, Y, and n are as described above] is, for example, a phosphorodiamidate morpholino oligomer (PMO) that is described in international publications (WO2001/083740, WO2006/000057, WO2010/048586, WO2010/050801, WO2011/057350, WO2014/144978, WO2014/153240, etc.) or can be recognized by those skilled in the art based on the description of these international publications, and is preferably a phosphorodiamidate morpholino oligomer (PMO) capable of skipping at least one selected from the group consisting of exons 51, 53, 45, 55, 44, and 50 of the human dystrophin gene, and specific examples thereof include Eteplirsen (see WO2006/000057, WO2010/050801, and WO2014/144978; CAS Registration Number: 1173755-55-9), Golodirsen (see WO2001/083740 and WO2006/000057; CAS Registration Number: 1422959-91-8), and Casimersen (see WO2001/083740, WO2006/000057, and WO2011/057350; CAS Registration Number: 1422958-19-7).

In one aspect of the present invention, an example of the morpholino nucleic acid oligomer obtained by the preparation method of the present invention is the morpholino nucleic acid oligomer of formula (I-2): 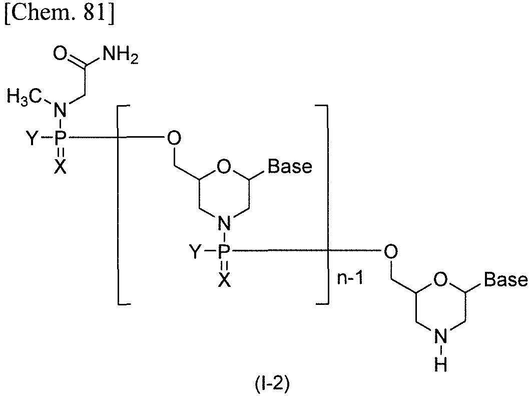 [wherein Base, X, Y, and n are as described above].

### [EXAMPLES]

### Reference Example 1: Morpholino monomer compounds

Table 2 shows the structural formulae and abbreviations of morpholino monomer compounds. As A^{p}, C^{p}, and T^{p}, commercially available products were used, or A^{p}, C^{p}, and T^{p} were prepared by methods commonly used in the art (see WO2008/008113). G^{CE} and G^{POB} were prepared by methods commonly used in the art (see WO2012/043730 and WO2009/064471).

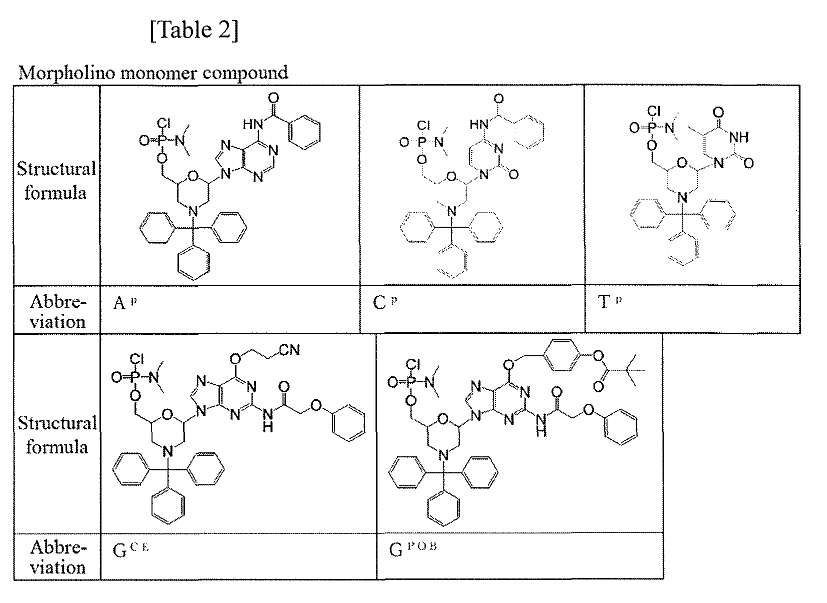

### Reference Example 2: Preparation of 4-{[(2S,6R)-6-(4-benzamido-2-oxopyrimidin-1-yl)-4-tritylmorpholin-2-yl]methoxy}-4-oxobutanoic acid

4-{[(2S,6R)-6-(4-Benzamido-2-oxopyrimidin-1-yl)-4-tritylmorpholin-2-yl]methoxy}-4-oxobutanoic acid was prepared by a method commonly used in the art (see WO2012/043730).

### Reference Example 3: Preparation of 4-{ [(2S,6R)-6-(4-benzamido-2-oxopyrimidin-1-yl)-4-tritylmorpholin-2-yl]methoxy}-4-oxobutanoic acid supported on aminomethyl polystyrene resin

10.0 g of an aminomethyl polystyrene resin (manufactured by WATANABE CHEMICAL INDUSTRIES, LTD.) was added to 130 mL of pyridine, 12.1 g of 4-{[(2S,6R)-6-(4-benzamido-2-oxopyrimidin-1-yl)-4-tritylmorpholin-2-yl]methoxy}-4-oxobutanoic acid prepared in Reference Example 2, 17.3 g of 1-ethyl-3-(3-dimethylaminopropyl)carbodiimide hydrochloride, and 2.7 g of 4-DMAP were added, and the mixture was washed with 10 mL of pyridine. Then, 15.3 mL of triethylamine and 20 mL of pyridine were added, and the mixture was stirred at room temperature for 1 day. After the reaction, the resin was filtered off. The obtained resin was washed with pyridine, dichloromethane, methanol, and tetrahydrofuran in this order. 125 mL of tetrahydrofuran, 15.6 mL of 2,6-lutidine, and 12.7 g of benzoyl chloride were added to the obtained resin, and the mixture was stirred at room temperature for 1 hour. The resin was filtered off, washed with tetrahydrofuran, methanol, and dichloromethane in this order, and dried under reduced pressure to obtain 13.6 g of the title compound.

The loading amount of the target product was determined by measuring a UV absorbance at 409 nm as the molar amount of trityl per gram of the resin using a known method. The loading amount of the resin was 550 µmol/g.

### UV measurement conditions

Instrument: U-2910 (Hitachi, Ltd.)
Solvent: methanesulfonic acid
Wavelength: 409 nm
ε value: 45000

### Example 1: Preparation of oligomer

Synthesis of morpholino nucleic acid oligomer whose base sequence is 5'-CCTCCGGTTCTGAAGGTGTTC-3'

### 1. Elongation reaction

3.0 g (1.7 mmol) of 4-{[(2S,6R)-6-(4-benzamido-2-oxopyrimidin-1-yl)-4-tritylmorpholin-2-yl]methoxy}-4-oxobutanoic acid supported on the aminomethyl polystyrene resin (Reference Example 3) was transferred into a reactor, and the synthesis cycle in Table 3 was initiated. The number of elongation reaction synthesis cycles was defined as the number corresponding to the chain length to be synthesized. In each condensation step, 1.3 to 2.2 molar equivalents of the morpholino monomer compound was used with respect to the oligomer obtained in the immediately previous elongation reaction synthesis cycle such that the base sequence of the morpholino nucleic acid oligomer was 5'-CCTCCGGTTCTGAAGGTGTTC-3'.

**[Table 3]**

| Details of elongation reaction synthesis cycle (Example 1) | | | | |
|---|---|---|---|---|
| Step | Process step | Reagent (solution) | Number of process step repetitions | Time/process step |
| 1 | Washing | Washing solution 1 | 1 | 5 minutes |
| 2 | Deprotection | Deprotection solution 1 | 4 to 7 | 5 minutes |
| 3 | Neutralization | Neutralization solution 1 | 3 | 5 minutes |
| 4 | Washing | Washing solution 2 | 2 | 5 minutes |
| 5 | Washing | Washing solution 3 | 2 | 5 minutes |
| 6 | Condensation | Condensation solution 1 (morpholino monomer compound + pretreatment solution 1) | 1 | 6 to 18 hours |
| 7 | Washing | Washing solution 2 | 1 | 5 minutes |
| 8 | Washing | Washing solution 1 | 2 | 5 minutes |

As a washing solution 1, 30% 2,2,2-trifluoroethanol (TFE)/dichloromethane (DCM) (containing 30% of TFE in DCM) was used.

As a washing solution 2, DCM was used.

As a washing solution 3, a pretreatment solution 1, as described below, was used.

As a deprotection solution 1, a solution in which a mixture of trifluoroacetic acid (1.2 equivalents) and triethylamine (1 equivalent) was dissolved in a DCM solution containing 1% (v/v) of triisopropylsilane (TIPS) and 20% (v/v) of TFE such that the concentration of the mixture was 5% (w/v) was used.

As a neutralization solution 1, a solution of isopropyl alcohol (IPA)/N,N-diisopropylethylamine (DIPEA)/DCM (ratio of 353:75:1065 (v/v)) was used.

As the pretreatment solution 1, a mixture of 1,3-dimethyl-2-imidazolidinone (DMI) containing N-ethylmorpholine (NEM) was used (5 to 6% NEM solution (v/v) in DMI).

As morpholino monomer compounds, A^{p}, C^{p}, T^{p}, and G^{CE} listed in Table 1 were used. A morpholino monomer compound was dissolved in the pretreatment solution 1 to prepare a 0.10 to 0.14 M morpholino monomer compound solution (condensation solution 1) in the pretreatment solution 1, and this solution was used as a condensation solution 1.

<Steps 5 to 7 in preparation of 18mer whose base sequence is 5'-CCTCCGGTTCTGAAGGTG-3'>

### Step 5

After Step 4, 25 mL of the washing solution 3 (5.3% NEM solution (v/v) in DMI) was added to the aminomethyl polystyrene resin on which a 17mer oligomer whose base sequence was 5'-CCTCCGGTTCTGAAGGT-3' was supported in the reaction vessel, the mixture was stirred for 5 minutes, and the solution in the reaction vessel was drained. Then, 25 mL of the washing solution 3 was added to the reaction vessel, the mixture was stirred for 5 minutes, and the solution was drained.

### Step 6

After Step 5, 25 mL of the condensation solution 1 (0.12 M G^{CE} solution in the pretreatment solution 1) was added to the reaction vessel, and the mixture was stirred for 18 hours.

### Step 7

After Step 6, the solution in the reaction vessel was drained. Then, 50 mL of the washing solution 2 was added to the reaction vessel, the mixture was stirred for 5 minutes, and the solution in the reaction vessel was drained.

### 2. Drying step

The aminomethyl polystyrene resin on which the morpholino nucleic acid oligomer synthesized above was supported was washed twice with DCM, collected from the reaction vessel, and dried under reduced pressure at 60°C for 6 hours. 14.6 g of the aminomethyl polystyrene resin on which the morpholino nucleic acid oligomer was supported was obtained.

### Example 2

### 1. Elongation reaction

3.0 g (1.5 mmol) of 4-{[(2S,6R)-6-(4-benzamido-2-oxopyrimidin-1-yl)-4-tritylmorpholin-2-yl]methoxy}-4-oxobutanoic acid supported on an aminomethyl polystyrene resin (synthesized in the same manner as in Reference Example 3) was transferred into a reactor, and the synthesis cycle in Table 4 was initiated. The number of elongation reaction synthesis cycles was defined as the number corresponding to the chain length to be synthesized. In each condensation step, 1.3 to 2.2 molar equivalents of the morpholino monomer compound was used with respect to the oligomer obtained in the immediately previous elongation reaction synthesis cycle such that the base sequence of the morpholino nucleic acid oligomer was 5'-CCTCCGGTTCTGAAGGTGTTC-3'.

In Example 2, the reactor was purged with nitrogen gas instead of Step 5 in Example 1. Specifically, after Step 4, the solution was drained, then a nitrogen balloon was attached to a lower portion of the filter of a filter reactor, a cock was opened, and nitrogen ventilation was performed twice.

**[Table 4]**

| Details of elongation reaction synthesis cycle (Example 2) | | | | |
|---|---|---|---|---|
| Step | Process step | Reagent (solution) | Number of process step repetitions | Time/process step |
| 1 | Washing | Washing solution 1 | 1 | 5 minutes |
| 2 | Deprotection | Deprotection solution 1 | 4 to 7 | 5 minutes |
| 3 | Neutralization | Neutralization solution 1 | 3 | 5 minutes |
| 4 | Washing | Washing solution 2 | 2 | 5 minutes |
| 5 | Condensation | Condensation solution 2 | 1 | 3 to 6 hours |
| 6 | Washing | Washing solution 2 | 1 | 5 minutes |
| 7 | Capping | Capping solution 1 | 1 | 10 minutes |
| 8 | Washing | Washing solution 2 | 1 | 5 minutes |
| 9 | Washing | Washing solution 1 | 2 | 5 minutes |

As a washing solution 1, 30% 2,2,2-trifluoroethanol (TFE)/dichloromethane (DCM) (containing 30% of TFE in DCM) was used.

As a washing solution 2, DCM was used.

As a deprotection solution 1, a solution in which a mixture of trifluoroacetic acid (1.2 equivalents) and triethylamine (1 equivalent) was dissolved in a DCM solution containing 1% (v/v) of triisopropylsilane (TIPS) and 20% (v/v) of TFE such that the concentration of the mixture was 5% (w/v) was used.

As a neutralization solution 1, a solution of isopropyl alcohol (IPA)/N,N-diisopropylethylamine (DIPEA)/DCM (ratio of 353:75:1065 (v/v)) was used.

As morpholino monomer compounds, A^{p}, C^{p}, T^{p}, and G^{CE} listed in Table 1 were used. A morpholino monomer compound was dissolved in a 5 to 6% DIPEA solution (v/v) in DMI to prepare a 0.10 to 0.13 M morpholino monomer compound solution (condensation solution 2), and this solution was used as a condensation solution 2.

As a capping solution 1, a mixture of acetic anhydride/DIPEA/DCM (ratio of 0.5:1.25:98.25 (v/v)) was used.

<Steps 2 to 7 in preparation of 18mer whose base sequence is 5'-CCTCCGGTTCTGAAGGTG-3'>

### Step 2

After Step 1, 45 mL of the deprotection solution 1 was added to the aminomethyl polystyrene resin on which a 17mer oligomer, whose base sequence was 5'-CCTCCGGTTCTGAAGGT-3' and which had a terminal protected with a trityl group, was supported in the reaction vessel, the mixture was stirred for 5 minutes, and the solution in the reaction vessel was drained. This step was further repeated five times.

### Step 3

After Step 2, 50 mL of the neutralization solution 1 was added to the reaction vessel, the mixture was stirred for 5 minutes, and the solution in the reaction vessel was drained. This step was further repeated twice.

### Step 4

After Step 3, 50 mL of the washing solution 2 was added to the reaction vessel, the mixture was stirred for 5 minutes, and the solution in the reaction vessel was drained. This step was further repeated once.

### Step 5

After Step 4, the reaction vessel was purged with nitrogen, 25 mL of the condensation solution 2 (0.12 M G^{CE} solution in a 5.3% DIPEA solution (v/v) in DMI) was added, and the mixture was stirred for 6 hours.

### Step 6

After Step 5, the solution in the reaction vessel was drained. Then, 50 mL of the washing solution 2 was added to the reaction vessel, the mixture was stirred for 5 minutes, and the solution was drained.

### Step 7

After Step 6, the solution in the reaction vessel was drained. Then, 50 mL of the capping solution 1 was added to the reaction vessel, the mixture was stirred for 10 minutes, and the solution was drained.

### 2. Drying step

The aminomethyl polystyrene resin on which the morpholino nucleic acid oligomer synthesized above was supported was washed three times with DCM, collected from the reaction vessel, and dried under reduced pressure at 60°C for 6 hours. 11.7 g of the aminomethyl polystyrene resin on which the morpholino nucleic acid oligomer was supported was obtained.

### Comparative Example 1: Preparation of oligomer

Synthesis of morpholino nucleic acid oligomer whose base sequence is 5'-CCTCCGGTTCTGAAGGTGTTC-3'

### 1. Elongation reaction

50 g (23 mmol) of 4-{ [(2S,6R)-6-(4-benzamido-2-oxopyrimidin-1-yl)-4-tritylmorpholin-2-yl]methoxy}-4-oxobutanoic acid prepared in the same manner as Reference Example 2 and supported on an aminomethyl polystyrene resin (loading amount of the resin was 458 µmol/g) was transferred into a reactor, and the synthesis cycle in Table 5 was initiated. In each condensation step, 2.0 to 3.0 molar equivalents of the morpholino monomer compound was used with respect to the oligomer obtained in the immediately previous elongation reaction synthesis cycle such that the base sequence of the morpholino nucleic acid oligomer was 5'-CCTCCGGTTCTGAAGGTGTTC-3'.

**[Table 5]**

| Details of elongation reaction synthesis cycle (Comparative Example 1) | | | | |
|---|---|---|---|---|
| Step | Process step | Reagent (solution) | Number of process step repetitions | Time/process step |
| 1 | Deprotection | Deprotection solution 2 | 3 to 8 | 5 minutes |
| 2 | Neutralization | Neutralization solution 2 | 4 to 5 | 2 minutes |
| 3 | Washing | Washing solution 2 | 3 | 1 minute |
| 4 | Washing | Washing solution 4 | 1 | 5 minutes |
| 5 | Condensation | Condensation solution 2 (morpholino monomer compound + pretreatment solution 2) | 1 | 3 to 8 hours |
| 6 | Washing | Washing solution 2 | 3 | 1 minute |
| 7* | Capping | Capping solution | 2 | 3 minutes |
| 8 | Washing | Washing solution 2 | 3 | 1 minute |

| | | | | |
|---|---|---|---|---|
| * The capping step was not performed in the final elongation reaction synthesis cycle. | | | | |

As a washing solution 2, DCM was used.

As a washing solution 4, a pretreatment solution 2, as described below, was used.

As a deprotection solution 2, a solution in which a mixture of trifluoroacetic acid (2 equivalents) and triethylamine (1 equivalent) was dissolved in a DCM solution containing 1% (v/v) of ethanol and 10% (v/v) of TFE such that the concentration of the mixture was 3% (w/v) was used.

As a neutralization solution 2, a solution of IPA/DIPEA/DCM (ratio of 25:5:70 (v/v)) was used.

As the pretreatment solution 2, a mixture of DIPEA/DMI (ratio of 10:90 (v/v)) was used.

As morpholino monomer compounds, A^{p}, C^{p}, T^{p}, and G^{CE} listed in Table 1 were used. A morpholino monomer compound was dissolved in the pretreatment solution 2 to prepare a 0.16 M morpholino monomer compound solution (condensation solution 2) in the pretreatment solution 2, and this solution was used as a condensation solution 2.

As a capping solution, a mixture of acetic anhydride/2,6-lutidine/DCM (ratio of 20:30:50 (v/v)) was used.

### 2. Drying step

The aminomethyl polystyrene resin on which the morpholino nucleic acid oligomer synthesized above was supported was collected from the reaction vessel, and dried under reduced pressure at room temperature for 2 days. 258.0 g of the aminomethyl polystyrene resin on which the morpholino nucleic acid oligomer was supported was obtained.

### Test Example 1: Comparison between morpholino nucleic acid oligomers prepared in Example 1 and Example 2 and morpholino nucleic acid oligomer prepared in Comparative Example 1

About 30 mg of each of aminomethyl polystyrene resins on which morpholino nucleic acid oligomers of Example 1 and Comparative Example 1 each obtained by synthesizing a morpholino nucleic acid oligomer, whose base sequence was 5'-CCTCCGGTTCTGAAGGTGTTC-3', by an elongation reaction and drying the morpholino nucleic acid oligomer, were supported was put in a reaction vessel, 0.75 mL of the deprotection solution 1 was added, and a reaction was carried out at room temperature for 40 minutes. The deprotection solution 1 was removed, and the resin was washed with the neutralization solution 1 and dichloromethane, and dried under reduced pressure at room temperature. 28% ammonia water-ethanol (4/1) was added to the dried resin, and the mixture was stirred at room temperature for 17 hours. The supernatant solution was diluted and subjected to HPLC (C18 ion-pair analysis) to measure the purity of the morpholino nucleic acid oligomer in the unpurified mixture.

By comparing the morpholino nucleic acid oligomers prepared in Example 1 and Example 2 with the morpholino nucleic acid oligomer prepared in Comparative Example 1, it was confirmed that the purity of the morpholino nucleic acid oligomer was improved by the preparation methods of Examples 1 and 2.

**[Table 6]**

| Purity of morpholino oligomer | |
|---|---|
| | Purity of morpholino nucleic acid oligomer |
| Example 1 | 68.5% |
| Example 2 | 62.9% |
| Comparative Example 1 | 57.5% |

### Test Example 2: Comparison between condensation reaction yields of morpholino nucleic acid oligomer prepared in Example 1 and morpholino nucleic acid oligomer prepared in Comparative Example 1

After each elongation cycle in 1. Elongation reaction in Example 1, a small amount of the aminomethyl polystyrene resin on which the morpholino nucleic acid oligomer was supported was taken, a 40% methylamine-methanol solution/water mixed solution was added, and a reaction was carried out at 65°C for 10 minutes to cleavage from the resin. Similarly, after each elongation cycle in 1. Elongation reaction in Comparative Example 1, a small amount of the aminomethyl polystyrene resin on which the morpholino nucleic acid oligomer was supported was taken, a 28% ammonia-water-ethanol mixed solution was added, and a reaction was carried out at 55°C overnight to cleavage from the resin. Each supernatant solution was diluted and subjected to HPLC (C18 ion-pair analysis), and the condensation reaction yield was calculated according to the following equation. Condensation reaction yield (%) = 100 x {1- peak area value of raw material*/(peak area value of raw material + main peak area value after condensation reaction)}

The raw material* refers to a morpholino nucleic acid oligomer having no trityl group at a terminal thereof and having a chain length shorter by one residue than the chain length obtained by the condensation reaction.

The condensation reaction yield of Example 1 was improved as compared to that of Comparative Example 1.

### <Abbreviations>

TFE: 2,2,2-trifluoroethanol
DCM: dichloromethane
TFA: trifluoroacetic acid
TEA: triethylamine
TIPS: triisopropylsilane
IPA: isopropyl alcohol
DIPEA: N,N-diisopropylethylamine
NEM: N-ethylmorpholine
DMI: 1,3-dimethyl-2-imidazolidinone

## Claims

1. A preparation method for a morpholino nucleic acid oligomer, comprising, in an elongation reaction of the morpholino nucleic acid oligomer: 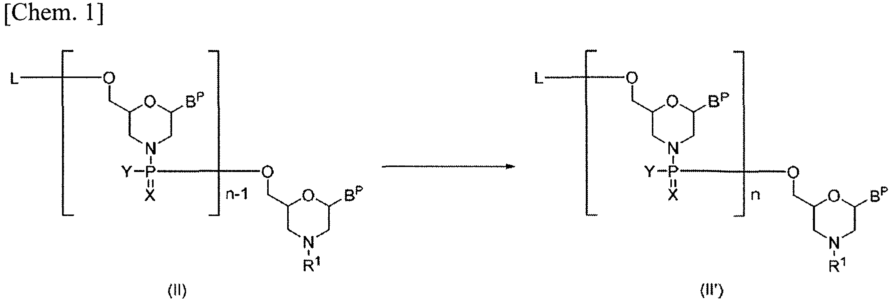 [wherein
each B^{P} is independently an optionally protected nucleic acid base,
R¹ is trityl, monomethoxytrityl, or dimethoxytrityl,
X is O or S,
Y is dialkylamino or alkoxy,
n is any integer in a range of 1 to 99, preferably any integer in a range of 15 to 30, and further preferably any integer in a range of 18 to 28, and
L is hydrogen, acyl, or a group represented by formula (IV): 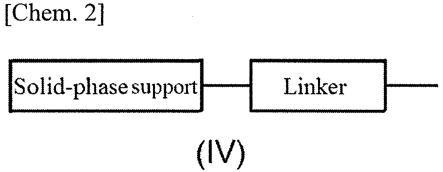 ],
a step of treating a compound of formula (II): 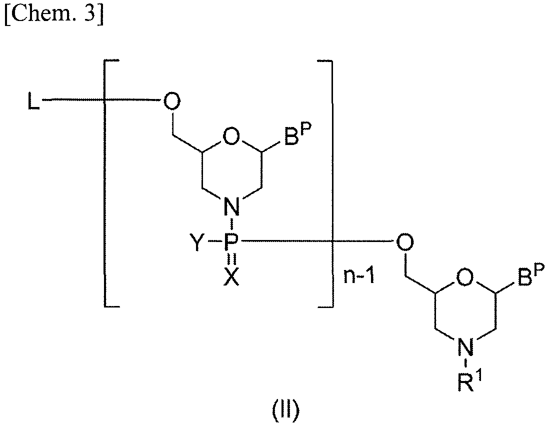
[wherein L, B^{P}, X, Y, R¹, and n are as described above]
with a solution containing an acid and a scavenger, to remove R¹ from the compound of formula (II) to obtain a compound of formula (III): 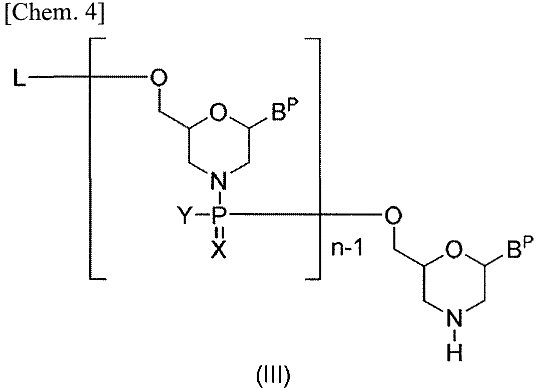
[wherein L, B^{P}, X, Y, and n are as described above].

2. The preparation method according to claim 1, further comprising a step of treating the compound of formula (III): 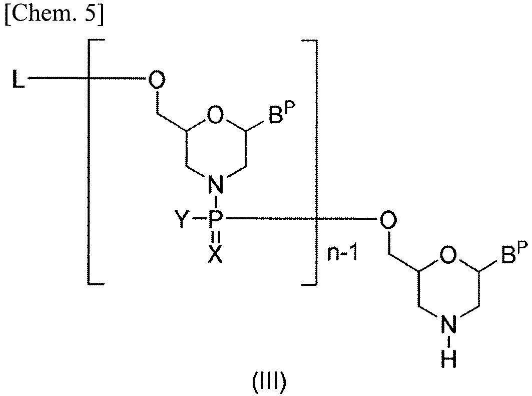
[wherein L, B^{P}, X, Y, and n are as described above]
with a solution containing a base, an alcohol, and a halogen solvent.

3. The preparation method according to claim 1 or 2, further comprising a step of treating the compound of formula (III): 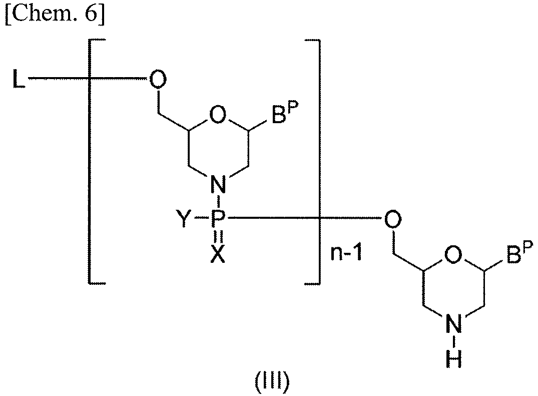
[wherein L, B^{P}, X, Y, and n are as described above]
with a solution containing an organic amine and an aprotic polar solvent.

4. The preparation method according to any one of claims 1 to 3, further comprising a step of treating a compound of formula (VIII): 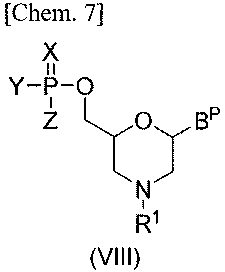
[wherein B^{P}, X, Y, and R¹ are as described above, and Z is a halogen]
with a solution containing an organic amine and an aprotic polar solvent.

5. The preparation method according to any one of claims 1 to 4, further comprising a step of reacting the compound of formula (III): 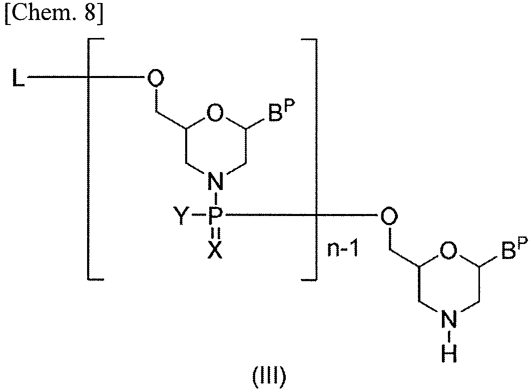
[wherein L, B^{P}, X, Y, and n are as described above]
in the presence of an organic amine with a compound of formula (VIII): 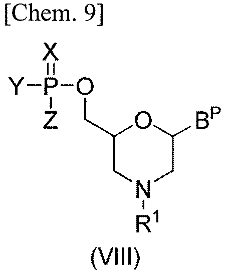
[wherein X, Y, Z, B^{P}, and R¹ are as described above]
to obtain a compound of formula (II'): 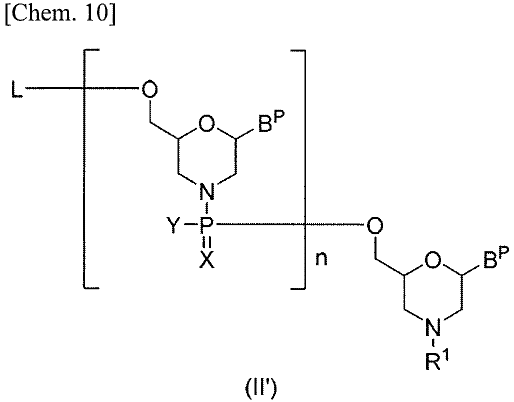
[wherein L, B^{P}, X, Y, R¹, and n are as described above].

6. The preparation method according to any one of claims 1 to 5, further comprising a step of treating a compound of formula (II'): 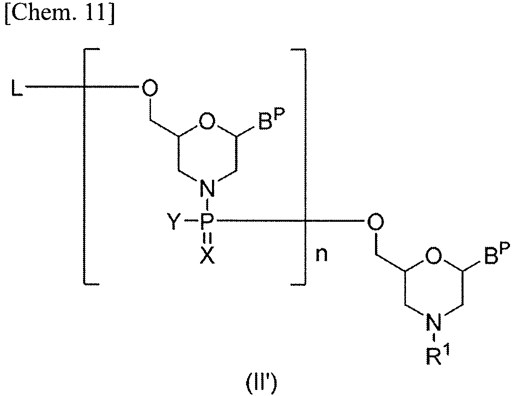
[wherein L, B^{P}, X, Y, R¹, and n are as described above]
with a solution containing an alcohol and/or a halogen solvent.

7. A preparation method for a morpholino nucleic acid oligomer, comprising, in an elongation reaction of the morpholino nucleic acid oligomer: 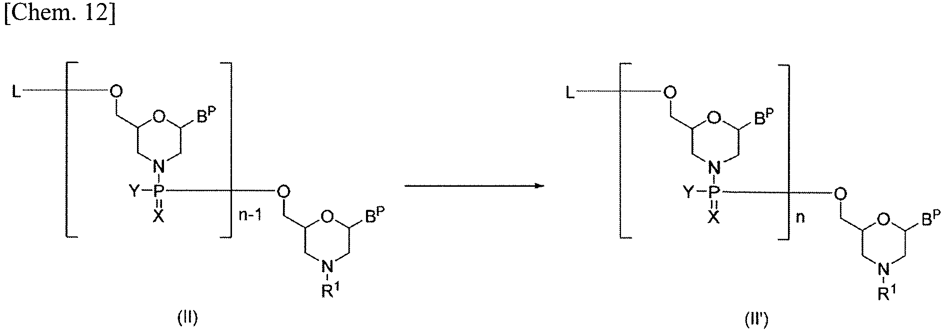 [wherein
each B^{P} is independently an optionally protected nucleic acid base,
R¹ is trityl, monomethoxytrityl, or dimethoxytrityl,
X is O or S,
Y is dialkylamino or alkoxy,
n is any integer in a range of 1 to 99, preferably any integer in a range of 15 to 30, and further preferably any integer in a range of 18 to 28, and
L is hydrogen, acyl, or a group represented by formula (IV): 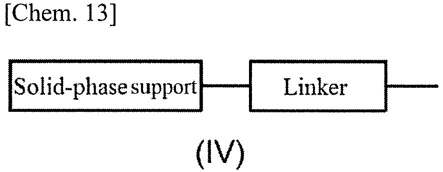 ],
a step of treating a compound of formula (II): 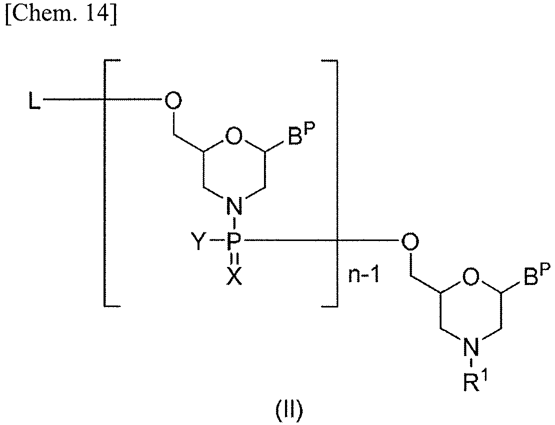
[wherein L, B^{P}, X, Y, R¹, and n are as described above]
with a solution containing an acid and a scavenger, to remove R¹ from the compound of formula (II) to obtain a compound of formula (III): 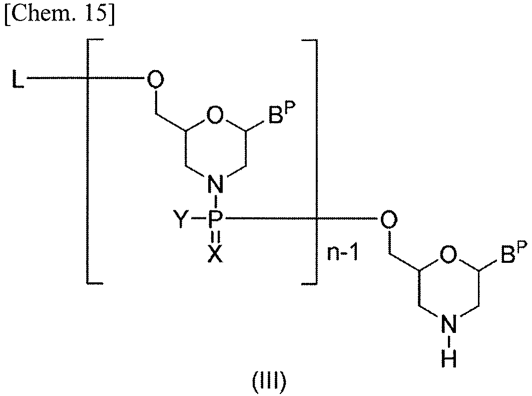
[wherein L, B^{P}, X, Y, and n are as described above],
a step of treating the compound of formula (III): 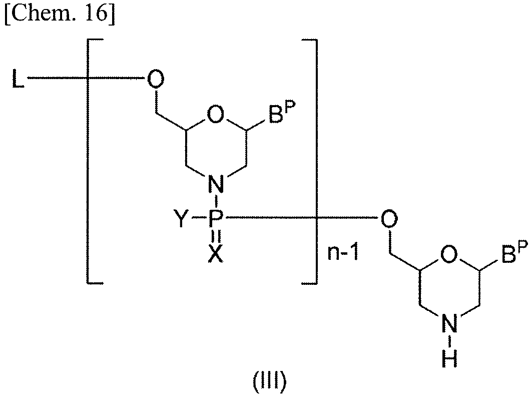
[wherein L, B^{P}, X, Y, and n are as described above]
with a solution containing an organic amine and an aprotic polar solvent, and
a step of treating a compound of formula (VIII): 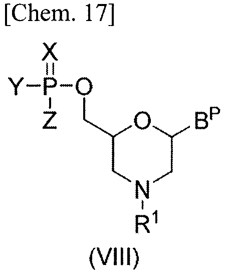
[wherein B^{P}, X, Y, Z, and R¹ are as described above]
with a solution containing an organic amine and an aprotic polar solvent.

8. A preparation method for a morpholino nucleic acid oligomer, comprising, in an elongation reaction of the morpholino nucleic acid oligomer: 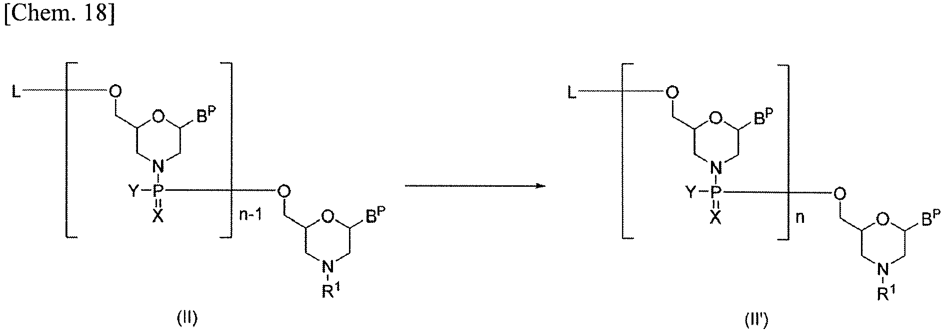 [wherein
each B^{P} is independently an optionally protected nucleic acid base,
R¹ is trityl, monomethoxytrityl, or dimethoxytrityl,
X is O or S,
Y is dialkylamino or alkoxy,
n is any integer in a range of 1 to 99, preferably any integer in a range of 15 to 30, and further preferably any integer in a range of 18 to 28, and
L is hydrogen, acyl, or a group represented by formula (IV): 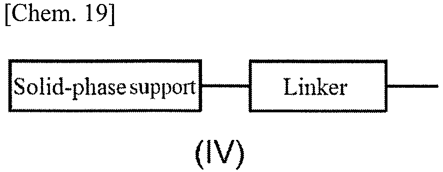 ],
a step of treating a compound of formula (II): 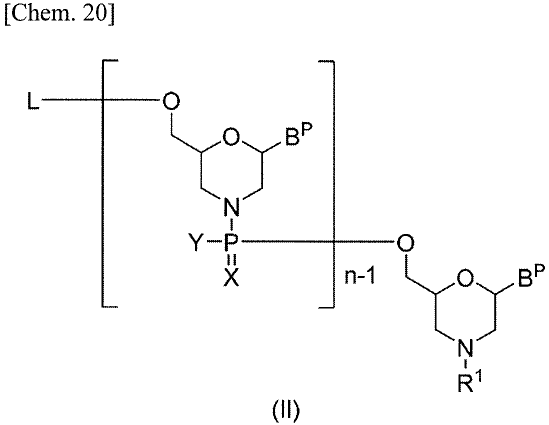
[wherein L, B^{P}, X, Y, R¹, and n are as described above]
with a solution containing an acid and a scavenger, to remove R¹ from the compound of formula (II) to obtain a compound of formula (III): 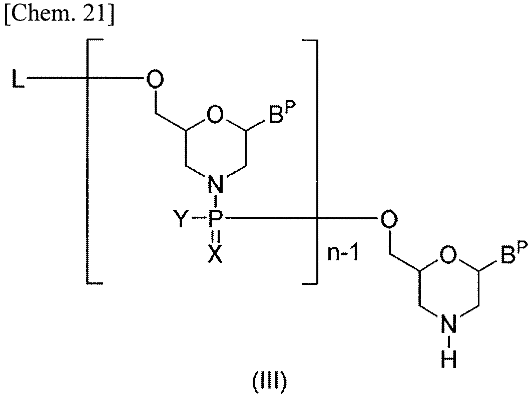
[wherein L, B^{P}, X, Y, and n are as described above], and
a step of treating the compound of formula (III): 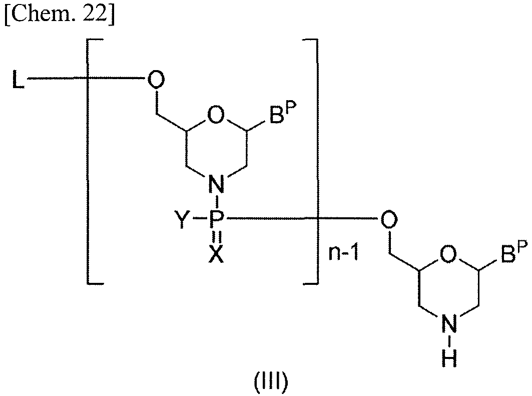
[wherein L, B^{P}, X, Y, and n are as described above]
and a compound of formula (VIII): 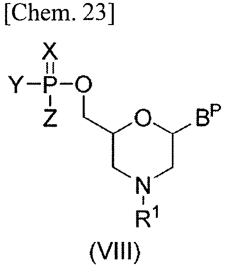
[wherein B^{P}, X, Y, Z, and R¹ are as described above, and Z is a halogen]
with a solution containing an organic amine and an aprotic polar solvent.

9. The preparation method for the morpholino nucleic acid oligomer according to any one of claims 1 to 8, wherein the solution containing the acid and the scavenger is a solution containing trifluoroacetic acid and triisopropylsilane.

10. The preparation method for the morpholino nucleic acid oligomer according to any one of claims 1 to 9, wherein the solution containing the acid and the scavenger is a solution containing trifluoroacetic acid, triethylamine, triisopropylsilane, 2,2,2-trifluoroethanol, and dichloromethane.

11. The preparation method for the morpholino nucleic acid oligomer according to any one of claims 1 to 9, wherein the solution containing the organic amine and the aprotic polar solvent is N-ethylmorpholine and 1,3-dimethyl-2-imidazolidinone.

12. The preparation method for the morpholino nucleic acid oligomer according to any one of claims 1 to 11, wherein the solid-phase support is a swellable polystyrene, a non-swellable polystyrene, a PEG chain-attached polystyrene, controlled pore glass, oxalylized controlled pore glass, a TentaGel support-amino polyethylene glycol-derivatized support, or a copolymer of Poros-polystyrene/divinylbenzene.

13. The preparation method for the morpholino nucleic acid oligomer according to any one of claims 1 to 12, wherein the linker is short-chain alkylene, long-chain alkylene, amino-short-chain alkylene, amino-long-chain alkylene, diacyl short-chain alkylene (e.g., succinyl), diacyl long-chain alkylene, or dialkylene sulfonyl.
